# EUROPEAN PATENT APPLICATION

(11) **EP 4 339 296 A1**
(43) Date of publication of application: **20.03.2024**
(21) Application number: 22382866.6
(22) Date of filing: 19.09.2022
(51) Int. Cl.: C12Q 1/48, G01N 33/50, G01N 33/574

(54) **METHOD FOR ASSESSING THE ACTIVITY OF NICOTINAMIDE N-METHYLTRANSFERASE**

(71) Applicant: Fundación para la Investigación Hospital Universitario y Politécnico La Fe de la Comunidad Valenciana, 46026 Valencia (ES); Universitat de València, 46010 Valencia (ES); Instituto de Investigación Sanitaria INCLIVA, 46010 Valencia (ES)
(72) Inventor: LAHOZ RODRÍGUEZ, Agustín, 46026 Valencia (ES); GARCÍA CAÑAVERAS, Juan Carlos, 46026 Valencia (ES); JUAN VIDAL, Oscar, 46026 Valencia (ES); HERVÁS, David, 46026 Valencia (ES); CARRETERO ASUNCIÓN, Julián, 46010 Valencia (ES); PULIDO ENDRINO, Inés, 46010 Valencia (ES); MENA MOLLA, Salvador, 46010 Valencia (ES); INSA MOLLÁ, Amelia, 46010 Valencia (ES); MARTÍN MARTORELL, Paloma, 46010 Valencia (ES)
(74) Representative: Hoffmann Eitle

(57) **Abstract**

The present invention refers to the use of specific metabolites for assessing the activity of NNMT, for selecting patients which may benefit from a treatment with NNMT inhibitors, for the prognosis and/or predicting the response of patients to NNMT inhibitors, for predicting whether a patient is resistant to a treatment with tyrosine kinase inhibitors and/or for screening NNMT inhibitor candidates.

## Description

### FIELD OF THE INVENTION

The present invention refers to the medial field. Particularly, the present invention refers to the use of specific metabolites for assessing the activity of nicotinamide N-methyltransferase (NNMT), for selecting patients which may benefit from a treatment with NNMT inhibitors, for the prognosis and/or predicting the response of patients to NNMT inhibitors, for predicting whether a patient is resistant to a treatment with tyrosine kinase inhibitors, for screening NNMT inhibitor candidates and/or the prognosis of cancer patients.

### STATE OF THE ART

In the U.S., up to 17% of Non-Small Cell Lung Cancer (NSCLC) patients harbor activating epidermal growth factor receptor (EGFR) kinase domain mutations that can be targeted with EGFR tyrosine kinase inhibitors (TKIs) to significantly improve overall survival. Despite drastic responses to the 1^{st} line gefitinib and erlotinib usually fails with acquired resistance, mediated primarily by the emergence of the secondary T790M genetic mutation or by focal amplification of the oncogene MET, in approximately 50% and 5% of patients, respectively. 3^{rd} generation EGFR TKIs that overcome the T790M-mediated EGFR-TKI resistance mechanisms have been used as 1^{st} and 2^{nd} line. However, most patients develop acquired resistance with various mechanisms including morphological transformation and epithelial to mesenchymal transition (EMT).

*In vitro,* NSCLC cells with an EMT phenotype are *de novo* resistant to EGFR TKIs despite the mutation of EGFR. EMT is a cellular program observed in normal development and is implicated in tumor progression and metastasis. Cancer cells presenting with a mesenchymal phenotype are poorly differentiated and often refractory to chemotherapy. It has been shown that the depletion of TGFβ1 or CXCR7 in the EGFR TKI resistant cells with a mesenchymal phenotype promotes mesenchymal to epithelial transition (MET) resulting in the re-sensitization of the cells to EGFR TKIs.

Nicotinamide N-methyltransferase (NNMT) promotes EMT program, leading to a high aggressiveness and short overall survival in cancer patients. So, there is an unmet medical need of finding strategies aimed at assessing NNMT activity with the objective of identifying patients with poor prognosis, selecting patients which may benefit from a treatment with NNMT inhibitors, for the prognosis and/or predicting the response of patients to NNMT inhibitors, for predicting whether a patient is resistant to a treatment with tyrosine kinase inhibitors and/or for screening NNMT inhibitor candidates.

The present invention is focused on solving this problem. Thus, specific metabolites, and combinations thereof, have been identified for that purpose.

### DESCRIPTION OF THE INVENTION

### Brief description of the invention

As explained above, the present invention refers to the use of specific metabolites, and combinations thereof, for assessing NNMT activity, for identifying patients with poor prognosis, for selecting patients which may benefit from a treatment with NNMT inhibitors, for the prognosis and/or predicting the response of patients to NNMT inhibitors, for predicting whether a patient is resistant to a treatment with tyrosine kinase inhibitors and/or for screening NNMT inhibitor candidates.

NNMT catalyzes the reaction between nicotinamide and S-adenosylmethionine (SAM) to produce 1-methylnicotinamide (1-MNA) and S-adenosylhomocysteine (SAH). NNMT consumes nicotinamide, limiting nicotinamide adenine dinucleotide (NAD+) content. Then, NNMT modulates NAD+, methionine and SAM pathways, impacting acetylation and methylation capacity in several diseases. NNMT expression is increased in neurodegenerative diseases such as Alzheimer's and Parkinson's diseases; hepatic diseases such as hepatitis, cirrhosis, fatty liver disease; atherosclerosis; pulmonary hypertension; obesity and metabolic syndrome; peripheral occlusive arterial disease; and chronic obstructive pulmonary disease; fibrosis; cancer or aging. Therefore, targeting NNMT or linked pathways may serve as a therapeutic strategy for treating a wide number of diseases [Motoaki Komatsu, Takeshi Kanda, Hidenori Urai , Arata Kurokochi, Rina Kitahama, Shuhei Shigaki, Takashi Ono, Hideo Yukioka, Kazuhiro Hasegawa, Hirobumi Tokuyama, Hiroshi Kawabe, Shu Wakino, Hiroshi Itoh. NNMT activation can contribute to the development of fatty liver disease by modulating the NAD + metabolism. Sci Rep 2018 Jun 5;8(1):8637. doi: 10.1038/s41598-018-26882-8]. In addition, the activity of NNMT was found to extend lifespan by decreasing cellular SAM levels, producing a starvation signal and consequently inducing autophagy. For example, inhibition of NNMT promotes myoblast differentiation *in vitro* and enhances fusion and regenerative capacity of muscle stem cells in aging models [Harshini Neelakantan, et al. Small molecule nicotinamide N-methyltransferase inhibitor activates senescent muscle stem cells and improves regenerative capacity of aged skeletal muscle. Biochem Pharmacol. 2019 May; 163:481-492*].*

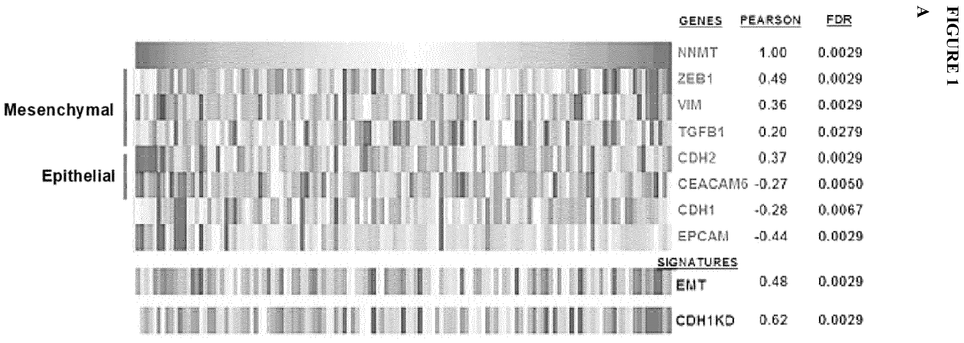

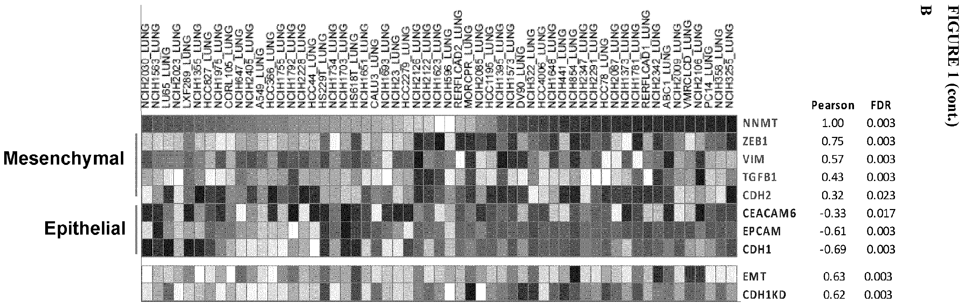

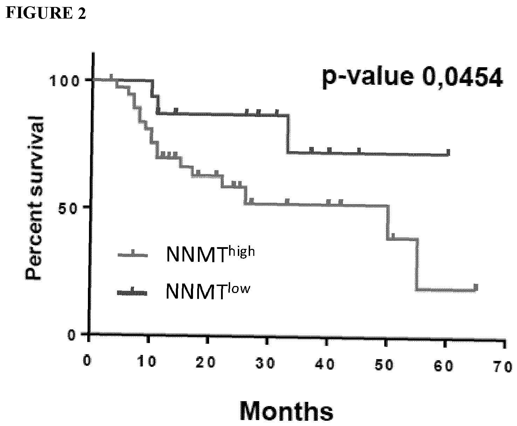

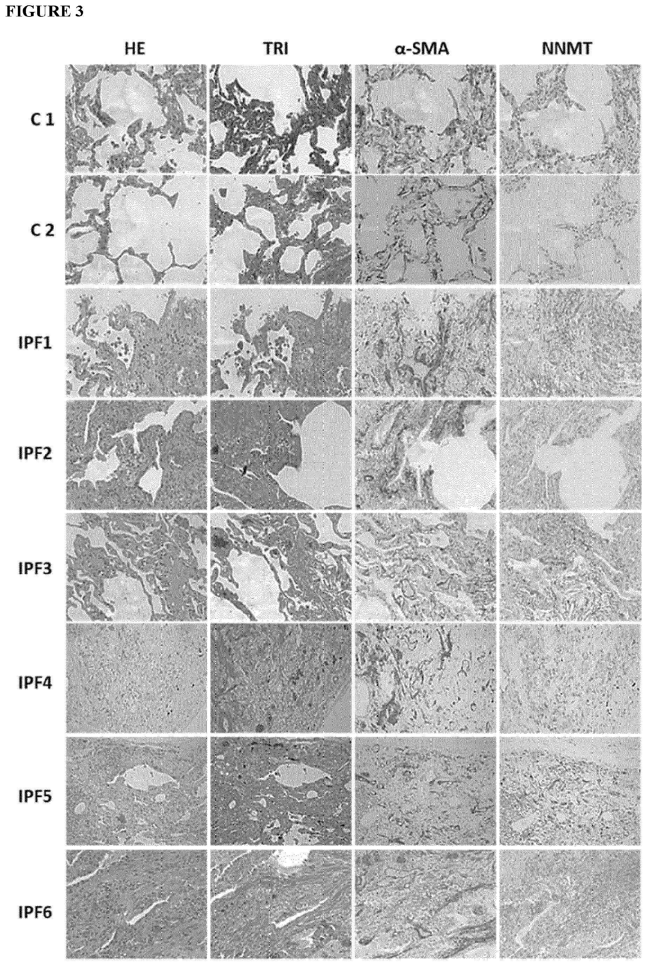

The results provided in the present invention indicate that the expression of NNMT positively correlates with a mesenchymal phenotype in lung tumor samples **(****Figure 1A****)** and lung tumor cell lines **(****Figure 1B**). Increased expression of *NNMT* is a poor prognosis factor independently of their histology, genotype or prior drug treatment **(****Figure 2****).** We have also observed NNMT is upregulated in Idiopathic pulmonary fibrosis (IPF) **(****Figure 3****),** the most common type of pulmonary fibrosis. Our data about correlation of expression of NNMT and high tumor progression and poor prognosis is consistent with evidence from other authors in other types of cancer [Dang L, Wang Y. Prognostic value of nicotinamide N-methyltransferase in human cancers: Evidence from a meta-analysis and database validation. Open Med (Wars). 2022;17(1):292-303. Published 2022 Feb 14. doi:10.1515/med-2022-0413*][* Wang W, Yang C, Wang T, Deng H. Complex roles of nicotinamide N-methyltransferase in cancer progression. Cell Death Dis. 2022 Mar 25;13(3):267. doi: 10.1038/s41419-022-04713-z. PMID: 35338115*].* NNMT expression also increase in BRAF mutated cancers relative to BRAF wild type [Ogawa, M., Tanaka, A., Namba, K. et al. Tumor stromal nicotinamide N-methyltransferase overexpression as a prognostic biomarker for poor clinical outcome in early-stage colorectal cancer. Sci Rep 12, 2767 (2022). https://doi.org/10.1038/s41598-022-06772-w*],* as well as in other diseases such as neurological diseases *[*Kocinaj A, Chaudhury T, Uddin MS, et al. High Expression of Nicotinamide N-Methyltransferase in Patients with Sporadic Alzheimer's Disease. Mol Neurobiol. 2021;58(4):1769-1781. doi:10.1007/s12035-020-02259-9*],* obesity or diabetes [Sampson, C.M., Dimet, A.L., Neelakantan, H. et al. Combined nicotinamide N-methyltransferase inhibition and reduced-calorie diet normalizes body composition and enhances metabolic benefits in obese mice. Sci Rep 11, 5637 (2021). https://doi.org/10.1038/s41598-021-85051-6].

The problem of resistance to therapy in cancer is complex. We propose an EMT process to drug resistance that are mediated by NNMT overexpression with metabolic and epigenetic reprogramming.

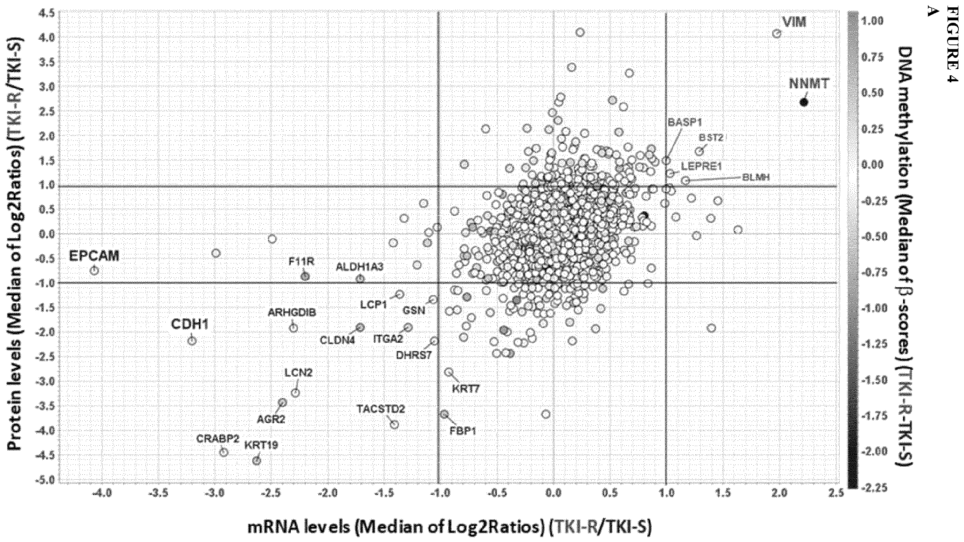

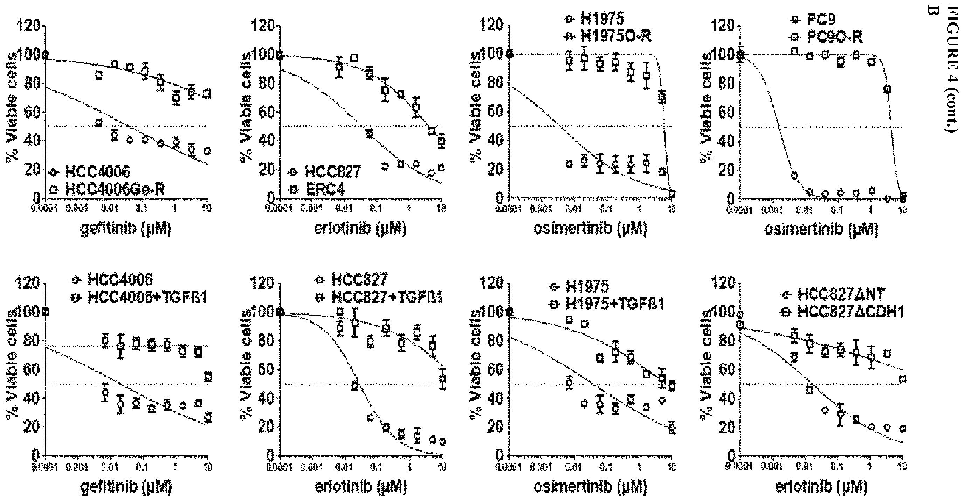

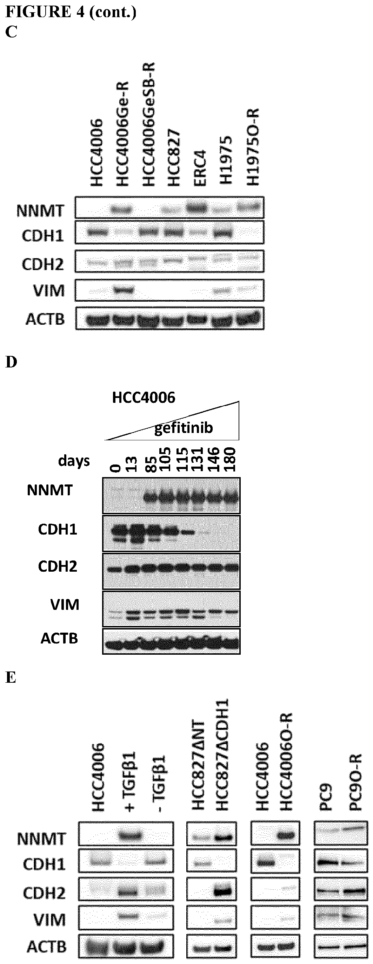

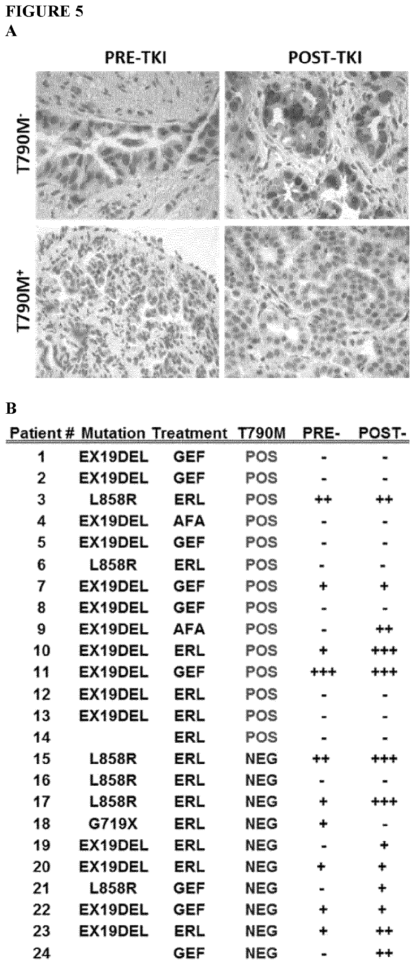

NNMT correlates with drug resistant acquisition levels in mesenchymal TKI-resistant clones via overexpression mediated by promoter hypomethylation **(****Figure 4A****).** We have observed in several TKI-resistant cell models **(****Figure 4B****)** that these resistant cells acquire a mesenchymal phenotype **(****Figure 4C-E****).** This data has been validated in a small cohort of paraffin-embedded lung tumor samples treated with TKIs **(****Figure 5****).**

The EGFR TKI-resistant mesenchymal cell models provided a platform to apply our multidimensional analyses to reveal *NNMT* as a significantly overexpressed gene under epigenetic regulation. Importantly, the resulting high NNMT protein levels in all analyzed EGFR TKI resistant NSCLC cell line models with a mesenchymal phenotype suggests strong correlation between mesenchymal phenotype and NNMT activity, likely offering a flexible metabolic adaptation to face the challenging tumor microenvironment exerted by drug treatments.

The link between EMT and resistance to treatment such as chemo or radiotherapy, immune- and targeted-therapies has been reported by other authors in different cancers [De Las Rivas, J., Brozovic, A., Izraely, S. et al. Cancer drug resistance induced by EMT: novel therapeutic strategies. Arch Toxicol 95, 2279-2297 (2021). htps://doi.org/10.1007/s00204-021-03063-7] including TKIs, but also MAPK inhibitors (e.g., RAS, RAF, MEK, ERK) [Padhye, Aparna et al. "Targeting CDK4 overcomes EMT-mediated tumor heterogeneity and therapeutic resistance in KRAS-mutant lung cancer." JCI insight vol. 6,17 e148392. 8 Sep. 2021, doi:10.1172/jci.insight.148392].

Like us, NNMT overexpression has been postulated as a mechanism in therapy-resistant disease against MAPK inhibitors [Eskiocak, B., McMillan, E. A., Mendiratta, S., Kollipara, R. K., Zhang, H., Humphries, C. G., Wang, C., Garcia-Rodriguez, J., Ding, M., Zaman, A., Rosales, T. I., Eskiocak, U., Smith, M. P., Sudderth, J., Komurov, K., Deberardinis, R. J., Wellbrock, C., Davies, M. A., Wargo, J. A., Yu, Y., ... White, M. A. (2017). Biomarker Accessible and Chemically Addressable Mechanistic Subtypes of BRAF Melanoma. Cancer discovery, 7(8), 832-851. https://doi.org/10.1158/2159-8290.CD-16-0955], or conventional chemotherapy as paclitaxel [Wang Y, Zeng J, Wu W, et al. Nicotinamide N-methyltransferase enhances chemoresistance in breast cancer through SIRT1 protein stabilization. Breast Cancer Res. 2019;21(1):64. Published 2019 May 17. doi:10.1186/s13058-019-1150-z].

To confirm the effect of NNMT expression on the sensitivity of TKI, we constructed several cell models in order to modulate the NNMT expression.

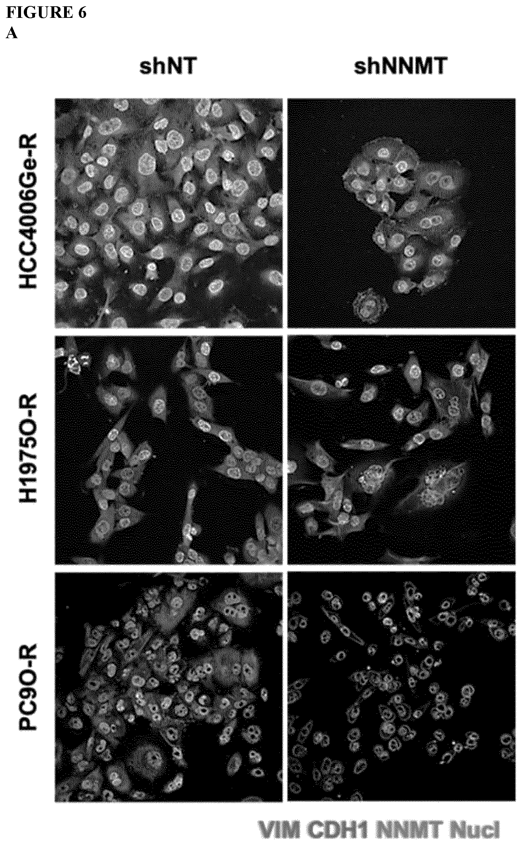

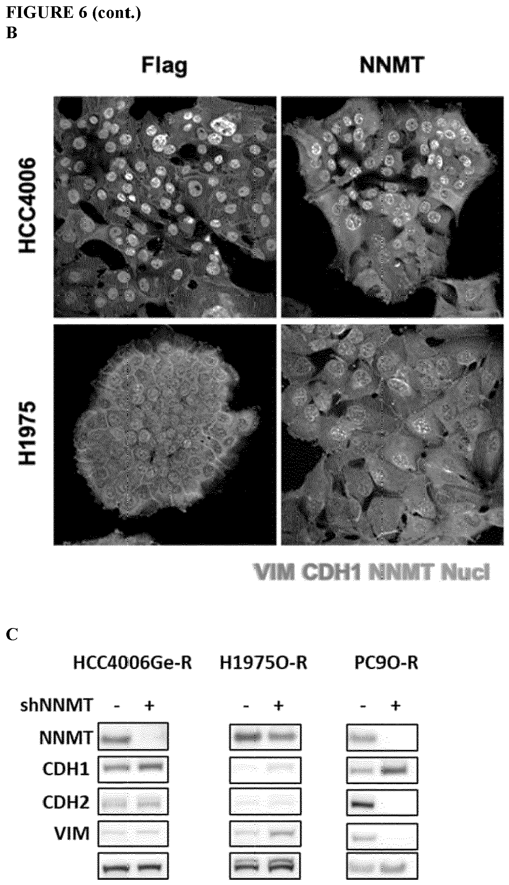

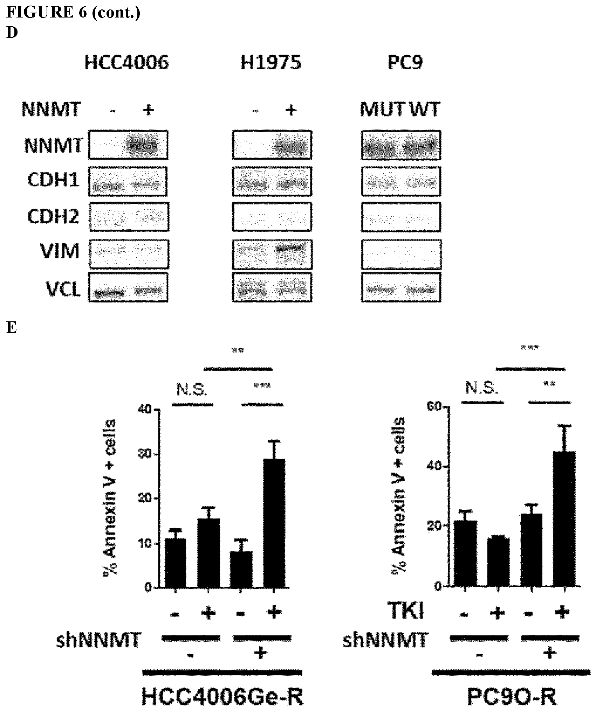

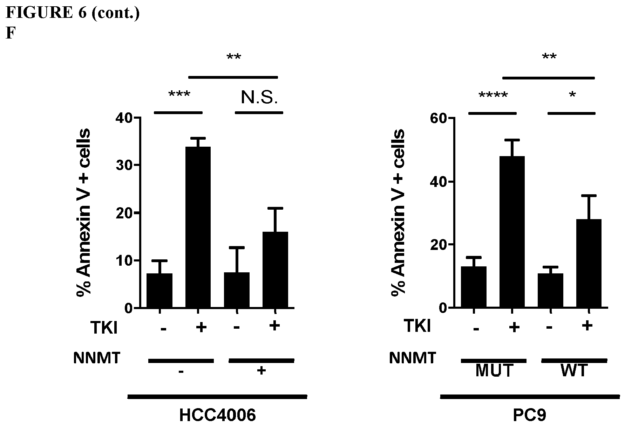

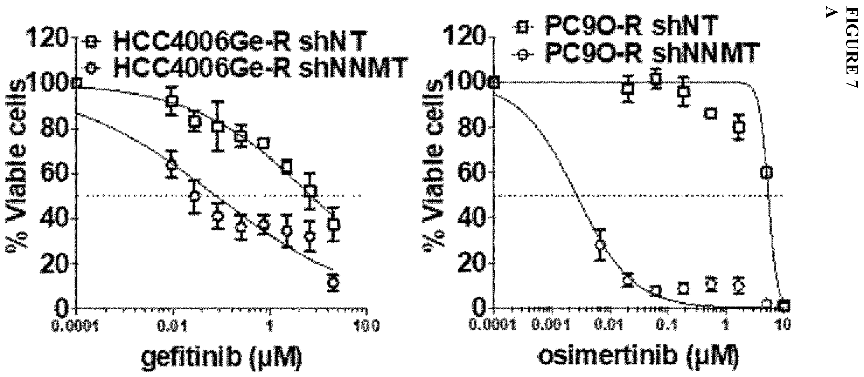

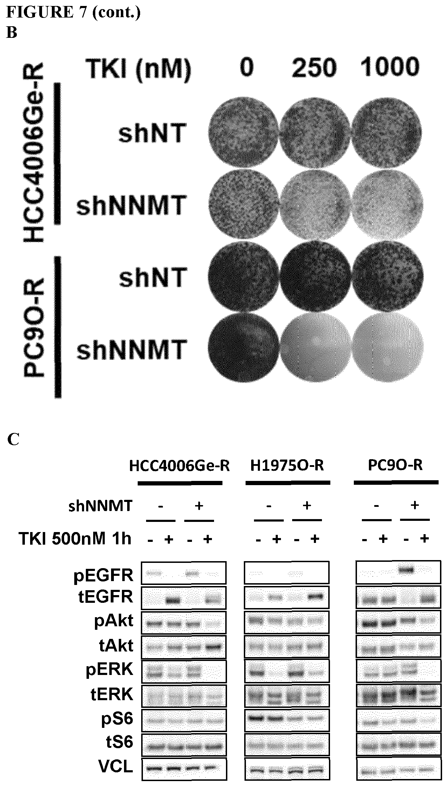

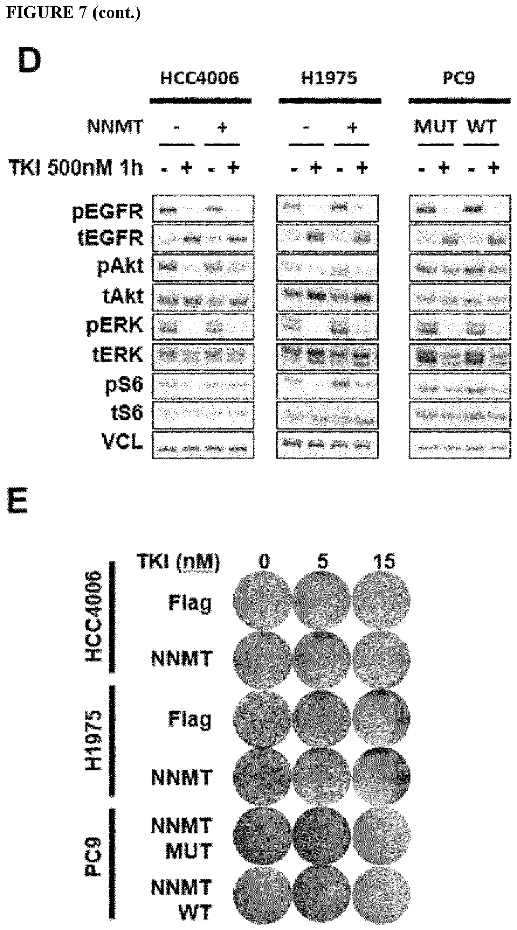

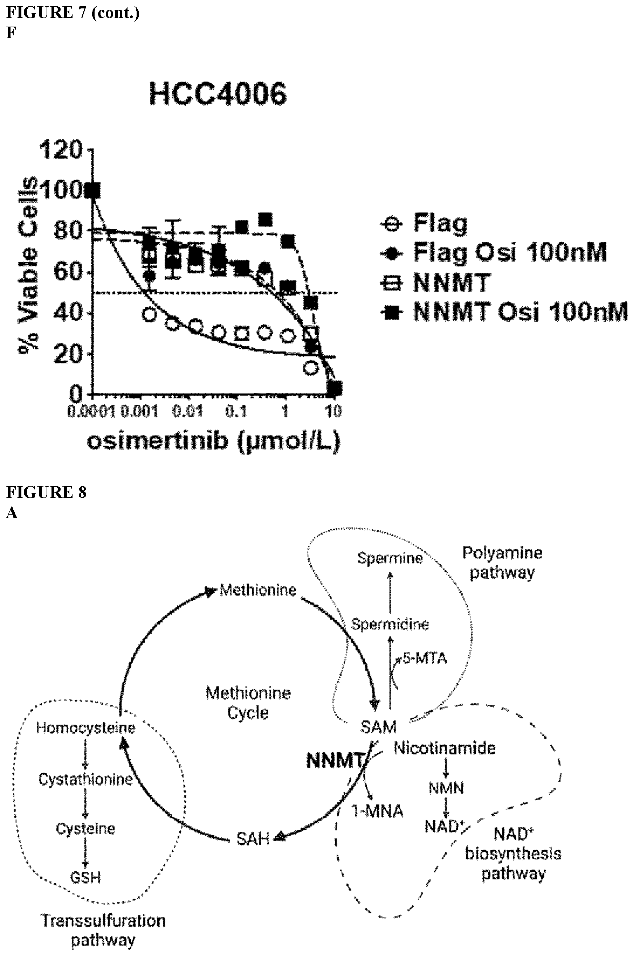

NNMT knocked down by Short Hairpin (sh) promoted EMT transition with reduced expression of canonical mesenchymal markers in EGFR mutant NSCLC cells grown resistant to EGFR TKIs **(****Figure 6A****,** **6C****,** **6E****,** **7A****,** **7B**, **7C****).** While genetic repression of NNMT by transfection with NNMT expression plasmid promoted MET phenotype with low levels of mesenchymal markers **(****Figure 6B**, **6D****,** **6F****,** **7D****,** **7E****,** **7F****).** Reduction of NNMT expression in EGFR TKI-resistant NSCLC restored the EGFR TKI sensitivity the cells to TKI. However, ectopic NNMT overexpression increased resistance to EGFR TKI. These results suggest that NNMT is necessary to maintain a mesenchymal phenotype but not enough to initiate EMT in EGFR mutant NSCLC cells, in EGFR mutant NSCLC cells, this mesenchymal state confers resistance to TKI's treatment and that pharmacological inhibition of NNMT could contribute to restoring TKI sensitivity.

Genetic repression of NNMT promoted MET **(****Figure 6A** **&** **6C****),** restoring the EGFR TKI sensitivity in the cells suggesting that pharmacological inhibition of NNMT could contribute to restoring TKI sensitivity **(****Figure 7A****)** in the subset of NSCLC with acquired resistance to EGFR TKIs with a mesenchymal phenotype.

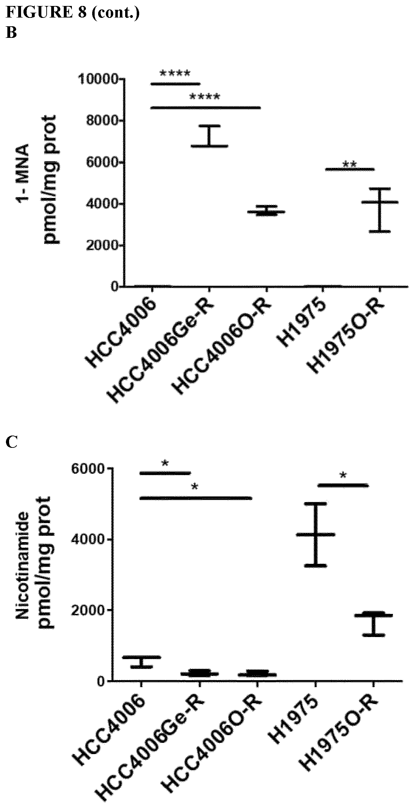

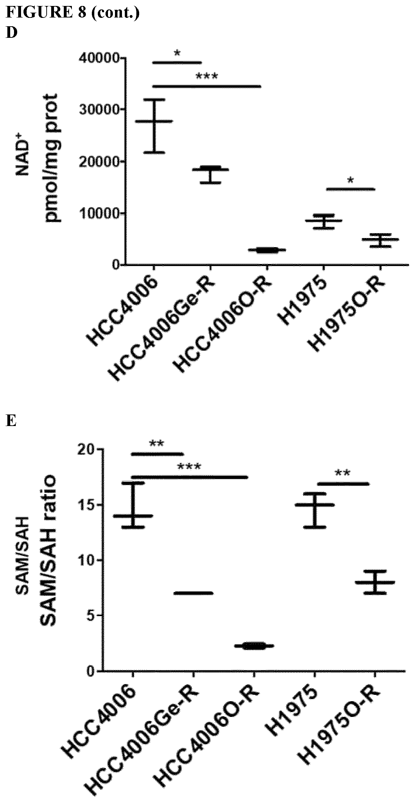

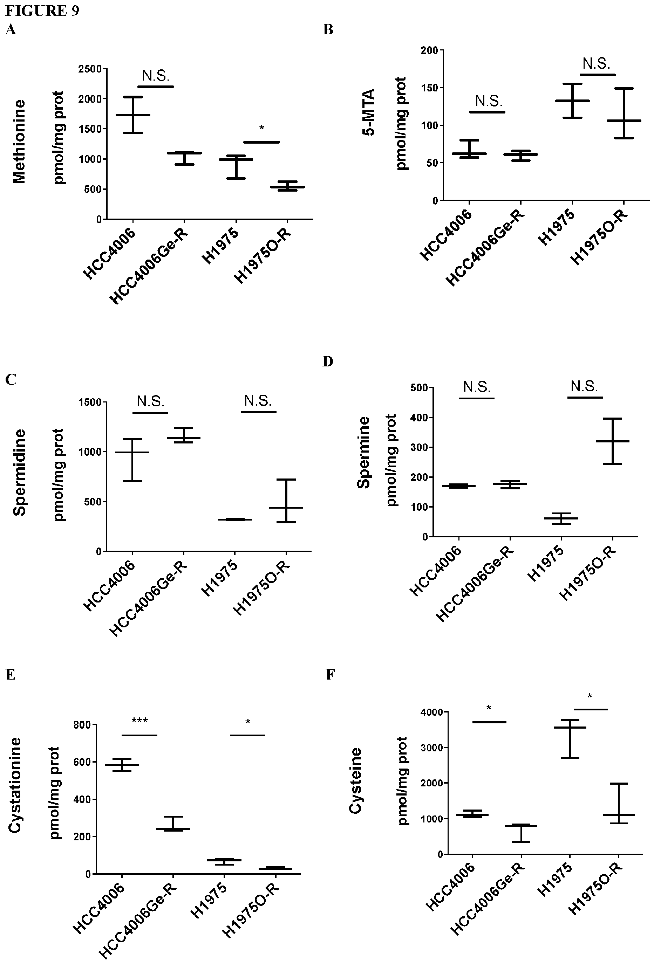

Additionally, we have observed a change in the profile of metabolites related to NNMT activity **(****Figure 8A****)** associated with this mesenchymal reprogramming. In our acquired EGFR TKI resistance models SAM methyl groups are transferred to nicotinamide (NAM) producing an increase in 1-MNA **(****Figure 8B****),** reducing Nicotinamide (NAM) levels **(****Figure 8C****)** and compromising NAD⁺ **(****Figure 8D****)** cellular pool in EGFR TKI-resistant NSCLC cells with a mesenchymal phenotype with increased NNMT expression. NNMT activity relies on an increased SAM consumption and SAH production, with a decrease in the SAM/SAH ratio **(****Figure 8E****).** SAM is replenished by methionine, so this metabolite decrease in TKI-resistant cell lines **(****Figure 9A****).** SAM sequestration by a higher NNMT activity produces changes in polyamines. On the other hand, low levels of SAH affect Homocysteine, a precursor of the trans-sulfuration pathway. In fact, metabolites involved in these pathways are altered **(Figure**

### 9B-H).

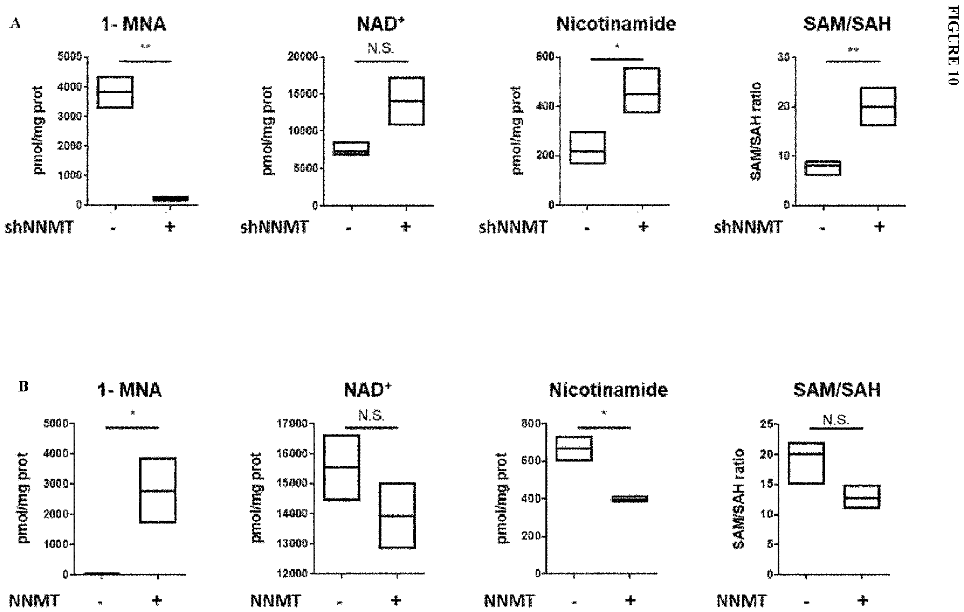

In order to verify the dependence of these metabolites on NNMT activity, we observed that HCC4006Ge-R9 resistant TKI cells, and therefore with an EMT phenotype and high levels of NNMT, modified with stably expressing shRNA targeting NNMT, decreased levels of 1-MNA. As NNMT activity decreased, the levels of nicotinamide, and its precursor NAD+, increased. SAM consumption and SAH production increased, as measured by the SAM/SAH ratio **(****Figure 10A****).** In contrast, with an exogen overexpressing NNMT on TKI-sensitive cells, such as the baseline HCC4006, we obtained an increased 1-MNA and SAH levels, and increased consumption of NNMT substrate Nicotinamide and NAD+ **(****Figure 10B****).**

These results demonstrate that: a) NNMT correlates with a mesenchymal phenotype; b) NNMT correlates with high aggressiveness and survival in NSCLC; c) NNMT correlates with resistance to cancer therapy; d) Actions aimed at reducing NNMT activity may induce an epithelial phenotype and sensitize to therapy; e) NNMT correlates with metabolites from methionine cycle, trans-sulphuration pathway, cystine/cysteine cycle, polyamine pathways and NAD+ salvage cycle, specially, 1-MNA;f) TKI-resistance correlates with metabolites from methionine cycle, trans-sulphuration pathway, cystine/cysteine cycle, polyamine pathways and NAD+ salvage cycle, specially, 1-MNA.

Moreover, NNMT overexpression removes nicotinamide, reducing NAD+ recycling, creating a vulnerability based on NAD⁺ depletion.

NAD⁺ is a fundamental coenzyme involved in energetic metabolism, DNA reparation or cell growth. Tumor cells show increased NAD⁺ synthesis to sustain their demanding needs and limiting NAD⁺ availability could counteract the metabolic changes supporting tumoral growth.

In several types of cancer have been described a high expression of Nicotinamide phosphoribosyl transferase (NAMPT) in order to maintain high NAD+. NAMPT has been associated to EMT, leading to a higher aggressiveness and shorter survival.

Clinical trials inhibiting NAMPT inhibition with Daporinad (FK866; DAP) demonstrate that they should be combined with other therapies to compensate the different NAD⁺ synthesis alternative pathways and expose the need for relevant biomarkers to select which tumors could be vulnerable.

Tumours with high expression of NNMT could be more susceptible to inhibitors of NAMPT, while low-NNMT expressing tumors might be poor responders to these NAMPT inhibitors. The remarkable drop in NAD⁺ levels in EGFR TKI-resistant cells with a mesenchymal phenotype **(****Figure 8D****)** can be increased by repressing NNMT **(****Figure 10A****)** and the results indicate an energetic vulnerability specific to the mesenchymal cells **(****Figure 11A****).** DAP selectively targets mesenchymal cells **(****Figure 11B**) with increased levels of NNMT. The increased apoptotic cell death **(****Figure 11F****)** is a direct consequence of the steep reduction in NAD⁺ **(****Figure 11C****)** and can only be counterbalanced by NNMT repression or inhibition **(****Figure 11E** **&** **11G****).** Inhibition of NAMPT by other experimental approaches such as the use of transient silencing with siRNAs against NAMPT (siNAMPT) also yielded the same results **(****Figure 12B****),** implying that inhibition of NAMPT can effectively be used in a systemic treatment to target mesenchymal TKI-resistant NNMT-driven tumors reducing significantly tumor volume.

These results demonstrate that: a) NNMT contributes to NAD+ pathway homeostasis; b) NAMPT inhibition targets a vulnerability specific to cells overexpressing NNMT; c) Inhibiting NAMPT seems then a useful approach when NNMT-mediated EMT cells become the predominant population in the tumor, while, alternative acquired EGFR-TKI resistance mechanisms not depending on an increase in NNMT expression, like MET amplification, are not sensitive to NAMPT inhibition; d) for all these reasons, identifying which patients present an increase in the NNMT pathway could serve to predict the response to NAMPT inhibitors. We have also shown that NNMT enzymatic activity can be inhibited by its product, 1-MNA, or by similar small-molecules like 1-methylquinolinium (1-MQ).

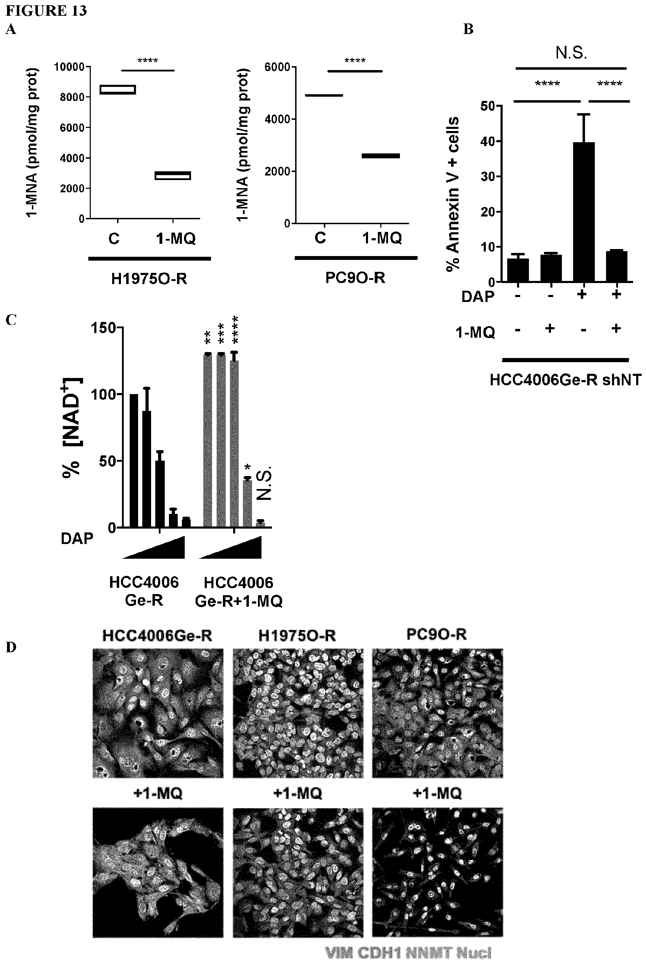

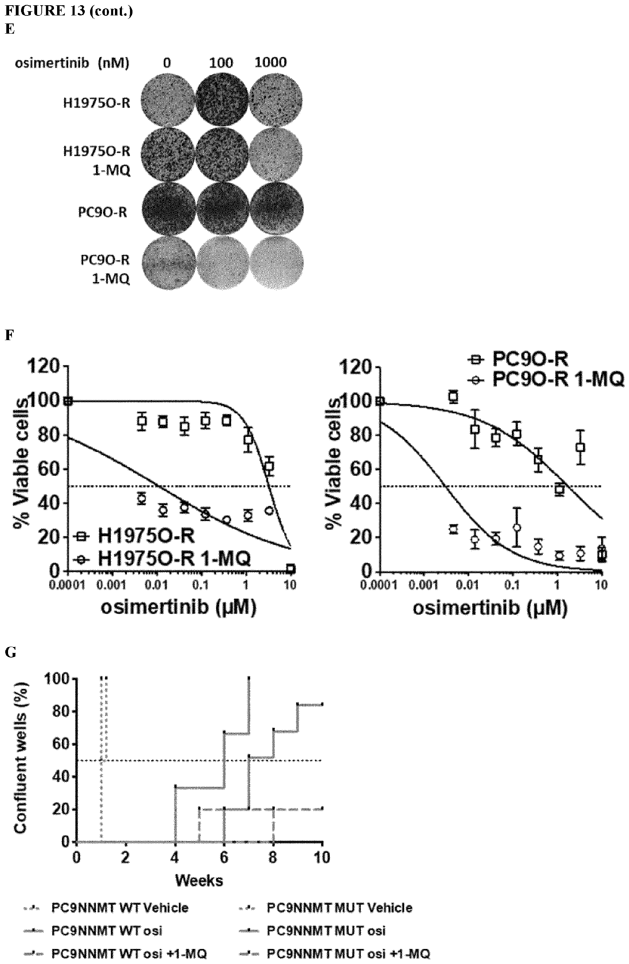

In our studies, treatment of EGFR TKI-resistant mesenchymal cells with 1-MQ resulted in a significant decrease in 1-MNA level that indirectly suggests a decreased NNMT activity **(****Figure 13A****).** We hypothesized that inhibition of NNMT activity with 1-MQ in mesenchymal resistant cells could prevent DAP cytotoxic effect. 1-MQ treatment abolishes completely DAP cytotoxicity in mesenchymal cells (Fig. 13B). This reduction in cell death can be explained because 1-MQ treatment prevented the abrupt drop of NAD+ levels (Fig. 13C) following DAP treatment in EGFR TKI-resistant with high expression of NNMT. Long-term treatment of EGFR TKI-resistant cells with 1-MQ promotes the return from mesenchymal cell to an epithelial state **(****Figure 13D****)** more sensitive to TKIs **(****Figure 13D** **&** **13F****).** Most importantly, in a NNMT- naive epithelial cell line, 1-MQ can prevent the emergence of NNMT and thus delaying the emergence of EMT resistant clones **(****Figure 13G****).**

Our results suggest that a) inhibiting NNMT activity would be more beneficial combined with TKI, before drug resistance appears, to prevent NNMT upregulation; b) combination of NNMT and NAMPT activity inhibition could be reduce aggressiveness and drug resistance appears, specially, in TKI therapies; and c) evaluating NNMT activity could be assist to identify which patients could benefit from these therapies.

In a similar way, NNMT could participate in regulating other NAD+-dependent enzymes. Sirtuins, are a family of deacetylases, regulate various cellular functions, including DNA repair, cell survival, and metabolism. They are altered in a broad of pathologies as cancer or aging. NNMT and its product MNA could increase Sirtuin stabilization and activity, leading to chemoresistance [Wang, Y., Zeng, J., Wu, W. et al. Nicotinamide N-methyltransferase enhances chemoresistance in breast cancer through SIRT1 protein stabilization. Breast Cancer Res 21, 64 (2019). https://doi.org/10.1186/s13058-019-1150-z]. So, our strategy could be used for these cases as well.

Based on the role of NNMT in physiopathology of several diseases and in our experience, the development of a method that allows evaluating the activity of NNMT could serve to study the EMT phenotype, study the resistance to therapy by mechanisms associated with EMT, study the sensitivity to therapies based on vulnerability based on NAD+ depletions, screening/studying/characterizing potential inhibitors of NNMT, screening/studying/characterizing potential inhibitors of NAMPT. In addition, NNMT can promote DNA and protein methylation disruption, implicated in different pathologies such as cancer. NNMT alters SAM levels, a methyl donor. And also, SAH levels and SAM/SAH ratio, a common indicator of cellular methylation potential. 1-MNA, NNMT's product, inhibits NAD+ synthesis, so may be an indicator of protein acetylation and ribosylation [Shlomi T, Rabinowitz JD. Metabolism: Cancer mistunes methylation. Nat Chem Biol. 2013;9(5):293-294. doi:10.1038/nchembio.1234].

Therefore, evaluating the activity of NNMT could be used to analyze activity of other enzymes implied in methylation or acetylation such as Sirtuins; methylthioadenosine phosphorylase (MTAP), an enzyme involved in the regeneration of SAM; glycine N-methyltransferase, which consumes SAM to methylate glycine; or others.

Due to the role of NNMT in several diseases, in recent years there has been increased interest in the development of NNMT inhibitors, such as SAM-competitive inhibitors, Nicotinamide-competitive inhibitors, Bisubstrate inhibitors (SAM and nicotinamide-binding pocket) or Covalent inhibitors [Yongzhi Gao, Nathaniel I Martin, Matthijs J van Haren. Nicotinamide N-methyl transferase (NNMT): An emerging therapeutic target. Drug Discov Today. 2021 Nov;26(11):2699-2706 Doi: 10.1016/j.drudis.2021.05.011],

However, to develop effective drugs directed against NNMT, it is necessary to have an assay for measuring NNMT activity, especially *in vivo.*

Assays for measuring NNMT activity involve the incorporation of radiolabeled 3H-methyl-SAM into the product 1-MNA, enzyme-coupled reactions to quantify the SAH by-product by fluorescence or luminescence, the condensation of 1-MNA with acetophenone results in the formation of fluorescent 2,7-naphthylpyridine analogs, or the use of 1-MQ as substrate for NNMT to measure the fluorescent change of reactions. However, these methods require external substrates, are not suitable for complex mixtures samples. Thus, specific assays for NNMT using liquid chromatography-mass spectrometry (LCMS)-based method for measuring the activity of NNMT have been developed [Gao Y, Martin NI, van Haren MJ. Nicotinamide N-methyl transferase (NNMT): An emerging therapeutic target. Drug Discov Today. 2021 Nov;26(11):2699-2706 doi: 10.1016/j.drudis.2021.05.011. Epub 2021 May 21. PMID: 34029690*].* These methods can be used to quantify simultaneously with very high specificity and sensitivity in complex samples the metabolic adaptations seen in the methionine cycle, trans-sulphuration pathway, cystine/cysteine cycle, polyamine pathways and NAD⁺ salvage cycle mediated by NNMT.

In view of the results suggesting the potential use of NNMT as a biomarker, the prognostic value of NNMT is still unclear and is currently not available for clinical practice, due in part to the ideal patient materials to diagnose, monitor and study the metabolic adaption of the disease are sequential fine needle biopsy samples from the same patient, which are rarely available and are not feasible to patients. Thus, liquid biopsies are an attractive approach to assess metabolic adaptation of the drug resistant tumors.

Interestingly, in order to obtain a non-invasive method to determine NNMT, its levels have been measured directly in blood or urine [Kim DS, Ham WS, Jang WS, Cho KS, Choi YD, Kang S, Kim B, Kim KJ, Lim EJ, Rha SY, Ku JH, Kwak C, Kim HH, Jeong CW, Cho NH Scale-Up Evaluation of a Composite Tumor Marker Assay for the Early Detection of Renal Cell Carcinoma. Diagnostics (Basel). 2020 Sep 25;10(10):750. doi: 10.3390/diagnostics10100750. PMID: 32992891; PMCID: PMC7601868] [Campagna R, Pozzi V, Spinelli G, et al. The Utility of Nicotinamide N-Methyltransferase as a Potential Biomarker to Predict the Oncological Outcomes for Urological Cancers: An Update. Biomolecules. 2021;11(8):1214. Published 2021 Aug 16. doi:10.3390/biom11081214], but there are few studies on this matter and the prognostic value of NNMT in blood is still unclear.

We have developed a method based on mass spectrometry that allows to evaluate the activity of NNMT in a high-output way with a high sensitivity. Furthermore, our method makes it possible to monitor NNMT activity in vitro and in vivo studies and in clinical samples from tumor or liquid biopsy.

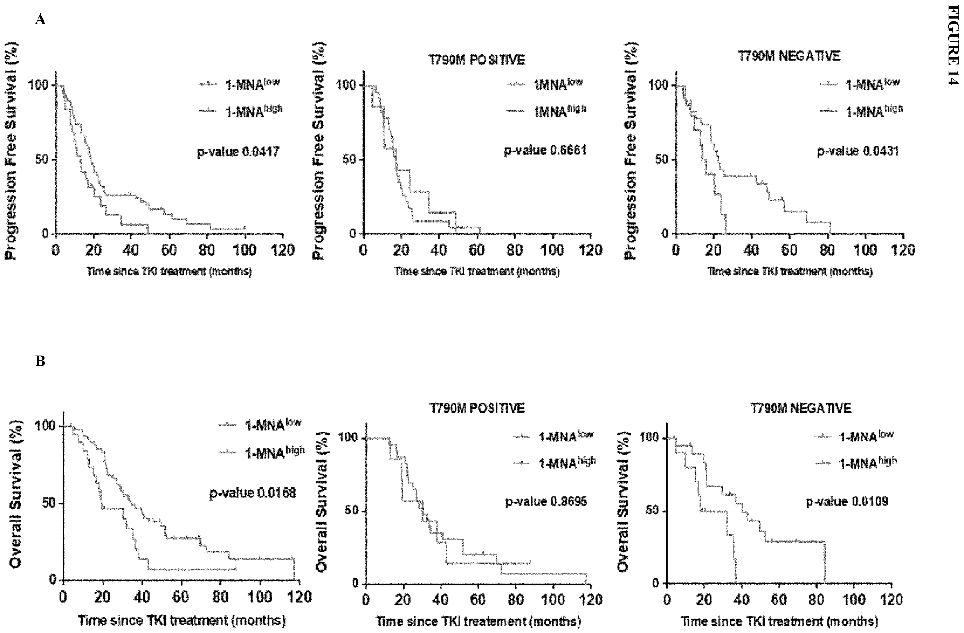

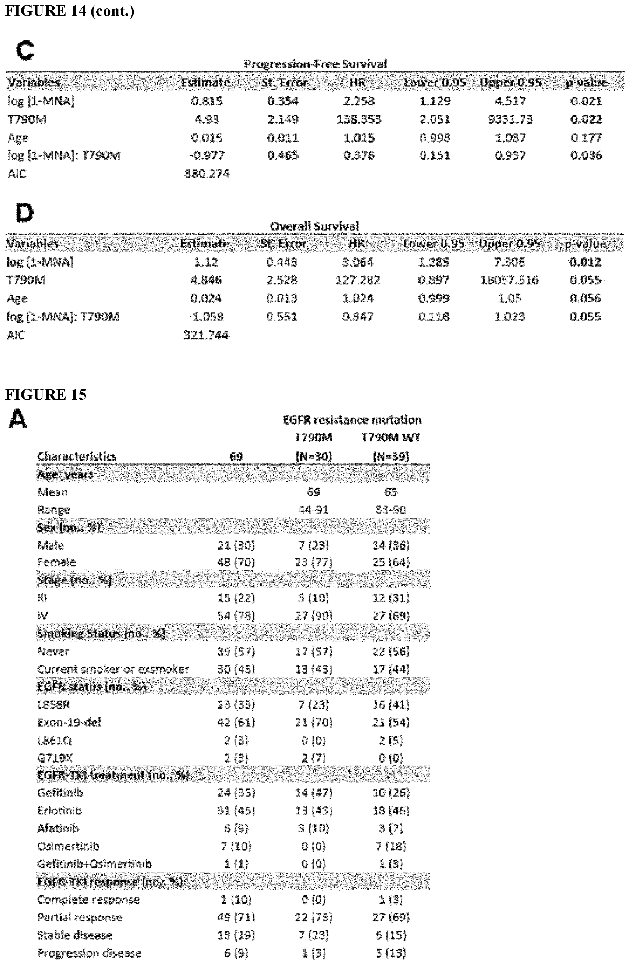

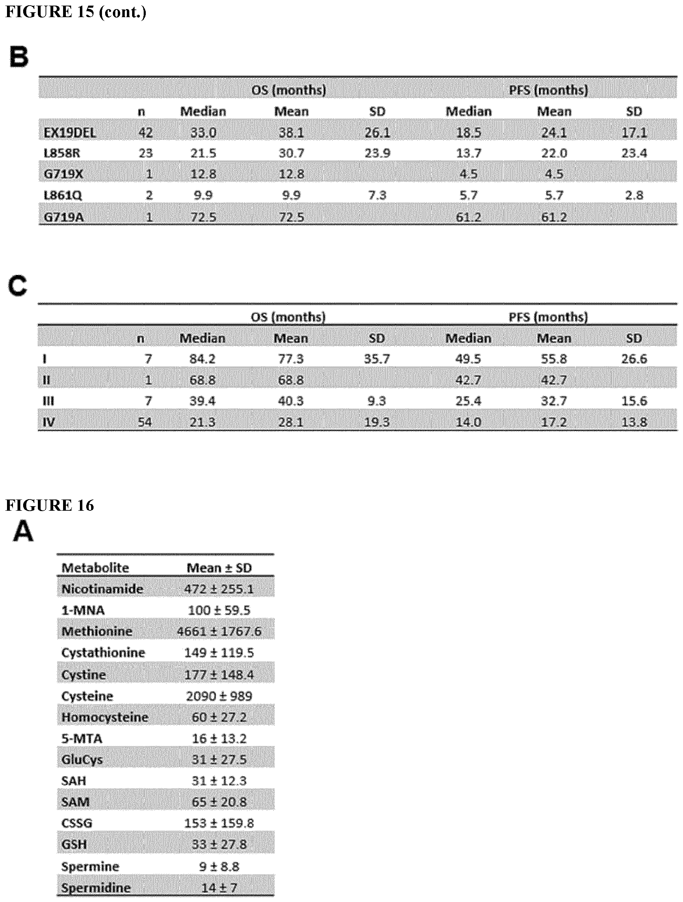

In our studies, 1-MNA levels correlates with NNMT levels in mesenchymal TKI-resistant clones **(****Figure 8A****)** and in NNMT overexpression and knock-down cellular models **(****Figure 10****).** It is important to highlight that, NNMT expression or plasma levels of its product MNA have already been proposed as biomarkers for these conditions [Kannt, A., Rajagopal, S., Kadnur, S. V. et al. A small molecule inhibitor of Nicotinamide N-methyltransferase for the treatment of metabolic disorders. Sci Rep 8, 3660 (2018). https://doi.org/10.1038/s41598-018-22081-7]. We measured 1-MNA as a surrogate of tumor NNMT activity in a less invasive sample as liquid biopsies from a cohort of 69 advanced EGFR-mutant NSCLC patients before they received EGFR TKIs. The median value of 1-MNA was used to stratify patients into high or low groups (i.e., above or below the median value). Basal 1-MNA was significantly associated with both progression-free survival (PFS) **(****Figure 14A****)** and overall survival (OS) **(****Figure 14B****),** where patients with higher levels had a significant shorter media. Moreover, multivariable data analysis revealed that 1-MNA and the T790M mutational status had a significant interaction as predictors of PFS (HR=0.376, 95% CI: 0.151-0937, p-value=0.03) and OS (HR=0.347, 95% CI: 0.118-1.023, p-value=0.04), suggesting an enrichment of mesenchymal-related TKI-resistance mechanism in the EGFR-T790M negative subgroup **(****Figure 14C & 14D****).** The Cox multivariable analysis did not show other significant associations with the remaining clinical variables **(****Figure 15****).** In summary, the increased NNMT, with high levels of 1-MNA, is significantly associated with EGFR mutant NSCLC tumors who progressed on EGFR TKIs without the T790M secondary mutation, pointing out NNMT activity as a predictive marker for both PFS and OS in patients. And furthermore, we have observed that combination of different biomarkers with 1-MNA may be a slightly better indicator than 1-MNA alone.

Considering that in addition to 1-MNA, methionine cycle, trans-sulphuration pathway, cystine/cysteine cycle, polyamine pathways or NAD⁺ salvage cycle are interrelated pathways. NNMT activity, EMT mechanism, prognosis and resistance treatment could be dependent on the balance between methyl groups from SAM and production of homocysteine (methionine cycle) to generate a substrate for NNMT the production of 1-MNA and its catabolism via NAD+ biosynthesis pathway; recycling of SAM by folate-dependent and -independent mechanisms or alternative catabolism of SAM via polyamine pathway; and homocysteine catabolism via the trans-sulphuration pathway.

In addition to 1-MNA, 14 distinguishable metabolites of these pathways were evaluated as candidate biomarkers: S-Adenosyl methionine (SAM), 5'-methylthioadenosine (5-MTA), Methionine, Cystine, Nicotinamide, Spermidine, Spermine, Cystathionine, Cysteine, HomoCysteine, Glutamyl-L-cysteine (GluCys), S-adenosyl-L-homocysteine (SAH), Glutathione Oxidized (CSSG), Glutathione (GSH) and Glutathione disulfide (GSSG).

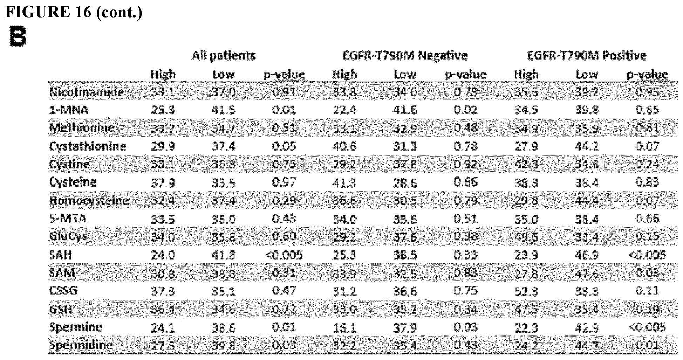

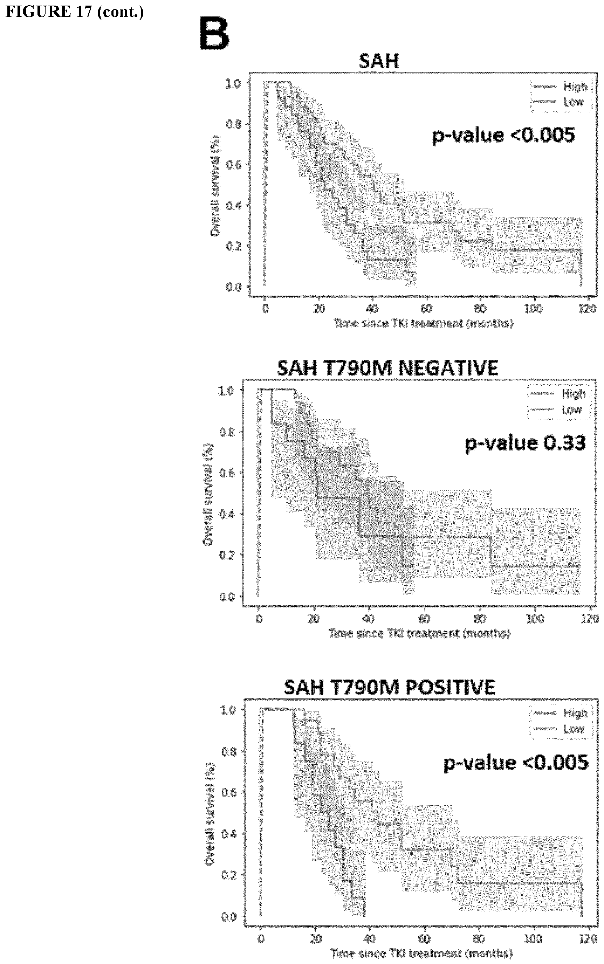

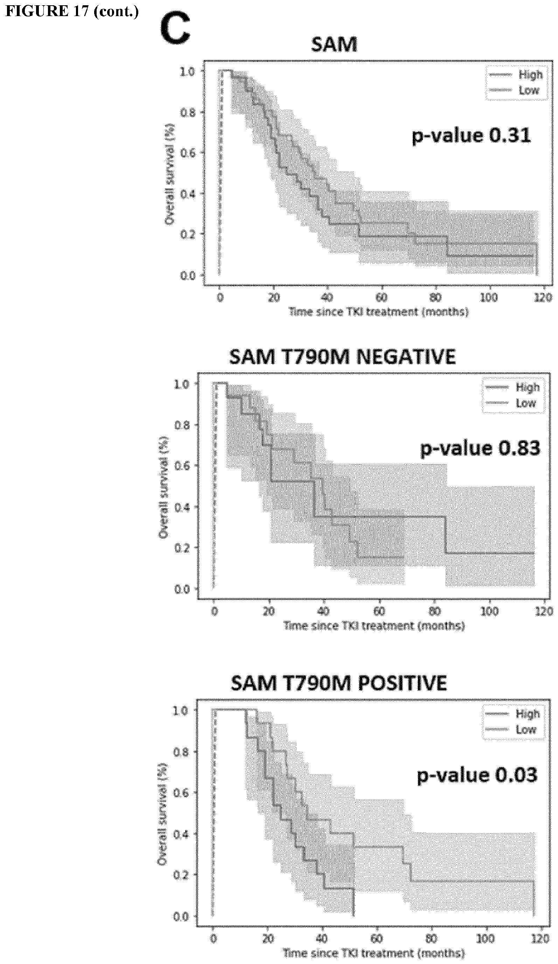

We measured these metabolites in liquid biopsies from a cohort of 69 advanced EGFR-mutant NSCLC patients before they received EGFR TKIs **(****Figure 16A****).** The mean value of each of them was used to stratify patients into high or low groups (i.e., above or below the median value). Only basal levels of 1-MNA, SAH, Spermine and Spermidine were significantly associated with OS **(****Figure 16B****).** Just like before, we evaluated interaction between metabolites and T790M mutational status. The results for 1-MNA were similar to those obtained by stratifying patients according to the median, showing a shorter survival for patients with EGFR-T790M negative subgroup and higher levels of 1-MNA **(****Figure 14** **&** **17B****).** We observed that patients with higher levels of SAH presented a shorter survival than patients with lower levels, with a mean of 24 .0 and 41.8 months respectively and a Log-Rank Test <0.005 (mean of OS for EGFR-T790M negative subgroup: SAH higher 25.3 SAH lower 38.5 months, Log-Rank Test 0.33; mean of OS for EGFR-T790M positive subgroup: SAH higher 23.9, SAH lower 46.9, Log-Rank Test <0.005) **(****Figure 16B** **&** **17B****),** indicating that SAH is a good marker to predict survival regardless of the presence or not of the EGFR-T790M mutation. This could be due to the fact that SAH, together with SAM, are a marker of the pool of methyl groups available for DNA and protein methylation. In this sense, we were able to observe that high levels of SAM correlate with lower survival in patients with EGFR-T790M mutation (mean of OS for EGFR-T790M positive subgroup: high levels of SAM 27.8, low levels of SAM 47.6 months, Log-Rank Test 0.03) **(****Figure 16B** **&** **17C****).**

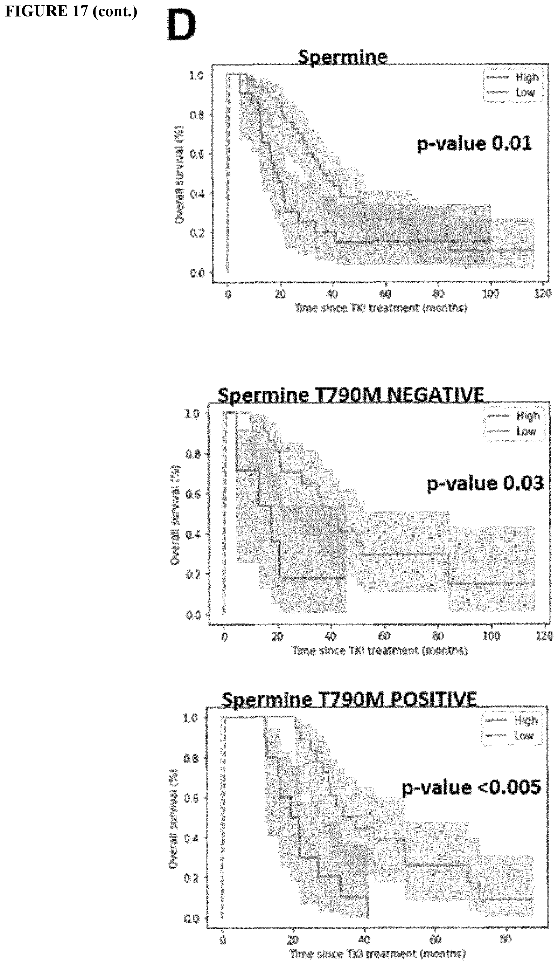

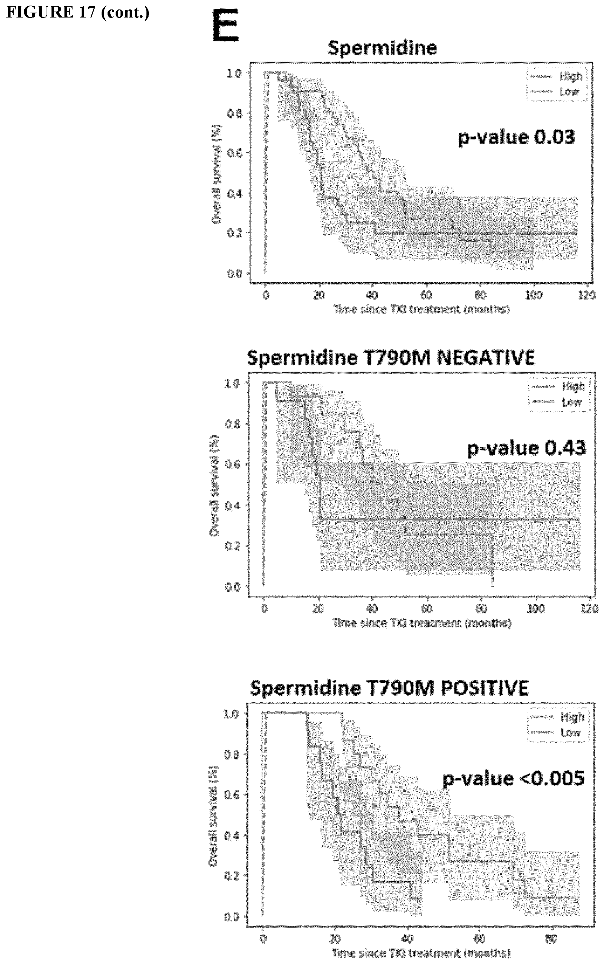

Spermine and Spermidine are polyamines synthesized from arginine, ornithine and methionine. They present positive charged, so interact with negatively charged like DNA and proteins, modulating chromatin structure and regulating gene expression [Muhammad, N.; Lee, H.M.; Kim, J. Oncology Therapeutics Targeting the Metabolism of Amino Acids. Cells 2020, 9, 1904.]. High levels of Spermine correlated with poor survival, regardless of the EGFR-T790M mutation, with 24.1 and 27.5 months for high and low levels subgroup respectively (p-value 0.01) **(****Figure 16B** **&** **17D****).** Spermidine showed a good negative correlation with survival in the EGFR-T790M positive mutation subgroup, with a median survival of 24.2 months in cases with high levels of Spermidine versus a median of 44.7 months in subgroup with low levels (Figure 16B **&** **17E****).**

In summary, the increased NNMT, with high levels of 1-MNA, is significantly associated with EGFR mutant NSCLC tumors who progressed on EGFR TKIs without the T790M secondary mutation, pointing out NNMT activity as a predictive marker for both PFS and OS in patients. Moreover, these data suggest that the analysis of different metabolites associated with NNMT activity could be useful for predicting TKI-resistance, regardless of the existence of other resistance mechanisms such as the acquisition of the EGFR-T790M mutation. Furthermore, we have observed that combination of different biomarkers with 1-MNA may be a slightly better indicator than 1-MNA alone.

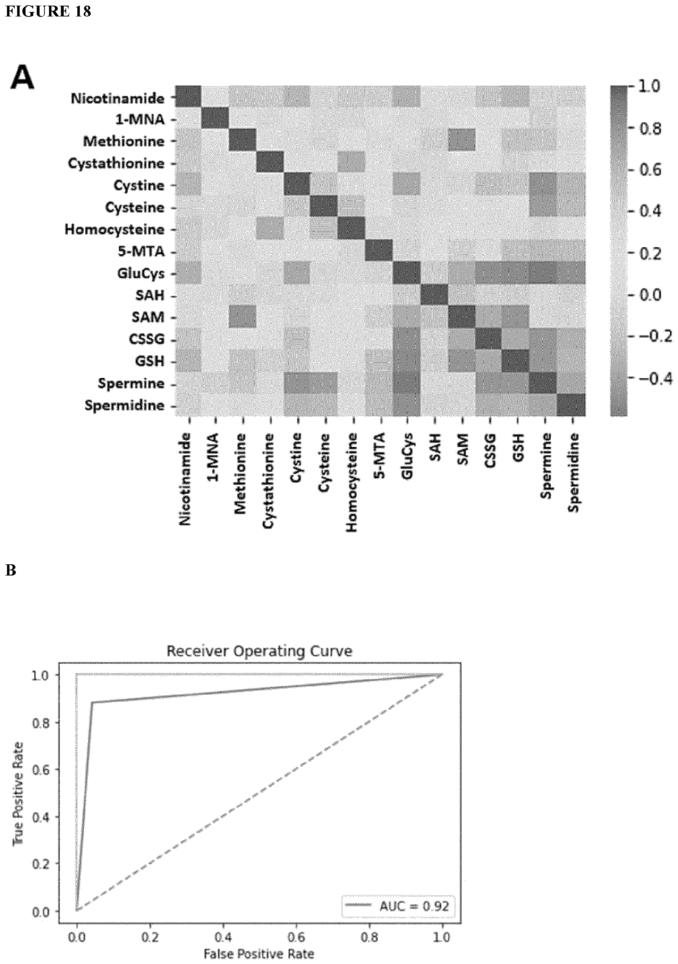

Before proceeding to investigate the possibility of obtaining risk prediction models using different biomarkers and clinical variables, we proceeded to evaluate the possible existence of correlation among predictors that could harm the models. No significant correlation was detected between the metabolites individually, only a weak positive correlation was detected between the levels of GSH and GluCys, with an r of 0.85 **(****Figure 18A****),** indicating the complexity of the metabolism and the multiple pathways in which each of them participates.

To test the hypothesis whether our selected metabolites related to NNMT are really related to each other and to the acquisition of resistance to TKIs, we used machine learning to define predictive algorithms. Machine learning methods are used extensively in medicine for screening, non-invasive diagnosis, post-treatment recurrence, and treatment selection. We generated a model for 1-MNA levels estimation from UPLC-MS/MS quantification of the other metabolites in blood. For this, we trained a model from the continuous data of the 14 metabolites against the previously established classification of High and Low for 1-MNA. We employed Gradient Boosting Machine (GBM), training a model that allowed to classify the samples correctly with an Area Under the Curve (AUC) of 0.92, with a sensitivity of 0.96 and specificity of 0.88 **(****Figure 18B****).**

Accurate prognosis assessment is essential for therapy selection in personalized medicine. However, there are currently no predictive tools available, so the choice of treatment remains empirical and with a high number of failures, mainly due to the acquisition of resistance. And even though the vast majority of NSCLC patients with somatic EGFR mutations show initial responses to treatment with TKIs, they develop or acquire resistance to these agents. Furthermore, there are several patients who do not respond to initial treatment with TKIs, and these patients are deemed to have primary or de novo resistance.

We have verified that the evaluation of NNMT activity could identify resistance mechanisms and predict in which cases a good response to TKI therapy is expected. We have also shown that NNMT activity can be assessed by analyzing 15 metabolites. We therefore aimed to establish a novel model to predict responses to TKIs using machine learning technique.

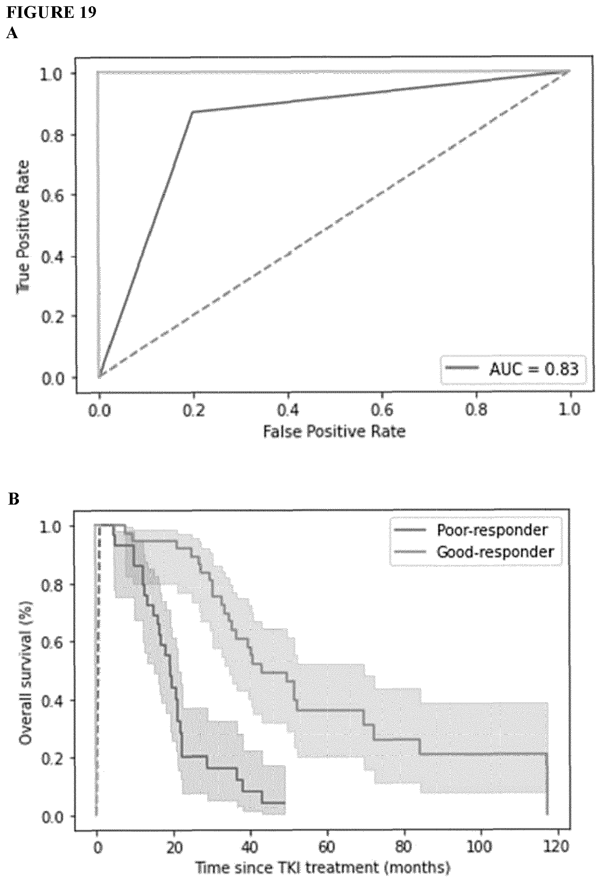

A gradient boosting machine (GBM) was trained to predict the likelihood of EGFR-mutated NSCLC patient's survival at 24 months after TKI treatment. GBM model predicted the response for TKI treatment with high accuracy (AUC: 0.83; Accuracy: 0.84; Sensitivity: 0.8; Specificity: 0.87) **(****Figure 19A****).** With this model, we classified patients as poor or good responders for TKI therapy and generated a Kaplan-Meier curve for OS, demonstrated that the model had high clinical utility. Our GBM model was able to stratify patients with a p-value <0.005 (Log-Rank test) **(****Figure 19B****).**

In our study, combined models significantly increased the prognostic performance of TKI response compared with individuals' analysis of metabolites, suggesting that panels of metabolites may be better than analysis of individuals metabolites.

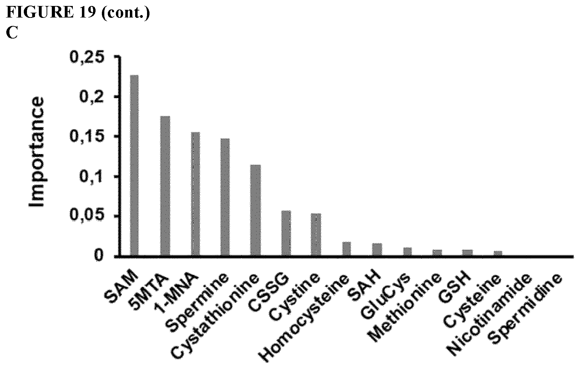

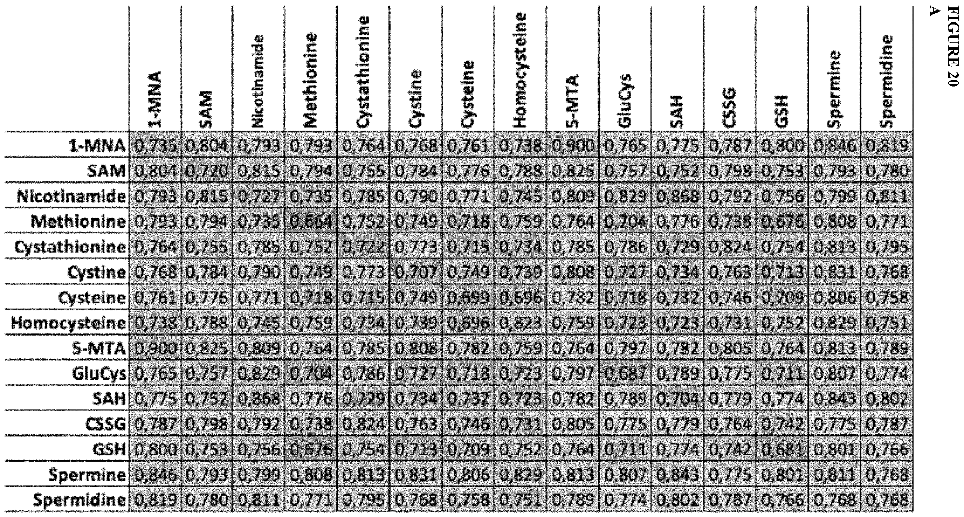

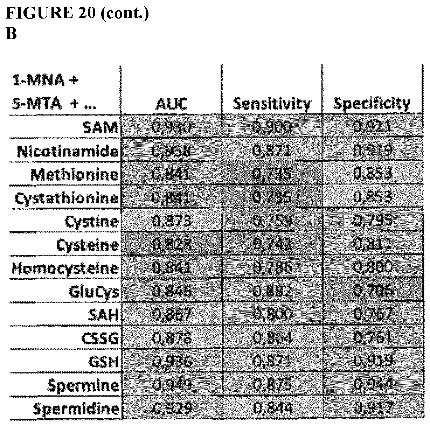

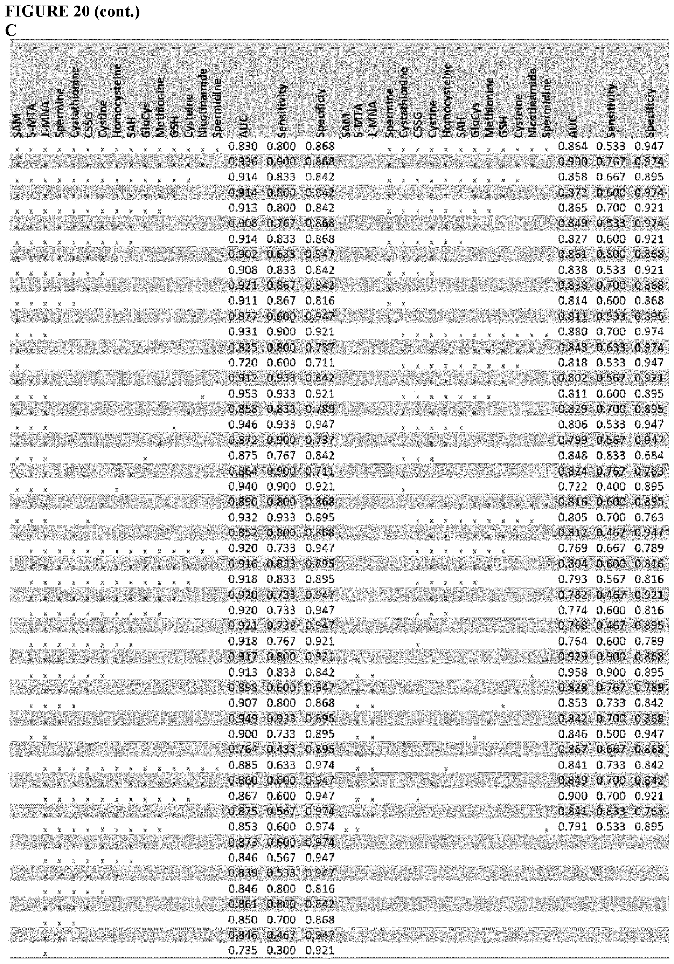

The metabolites variables have been ordered by importance score in the prediction of 24 months survival after TKI treatment **(****Figure 19C****).** We observed that 1-MNA appears in the first positions of the relevant variables list. Other variables in these positions were SAM, the most important methyl donor for NNMT and other epigenetic enzymes as DNA methyltransferases (DNMTs) or histone methyltransferases (HMTs); and 5-MTA, implied in SAM sequestration and therefore in NNMT activity. The polyamine pathway consumes decarboxylated SAM (AdoMetDC, generated from SAM by the action of SAM decarboxylase; SAMDC) and generates 5-MTA during the conversion of putrescine to spermidine and spermine. Note that these 3 metabolites are closely related to NNMT activity.
Different models using combinations of 15 metabolites were compared in terms of binary classifiers with AUC in ROC curves. We observed combination of 1-MNA, SAM and 5-MTA has a high predictive capacity. And additional metabolites to the model improved performance of the models **(****Figure 20****).**

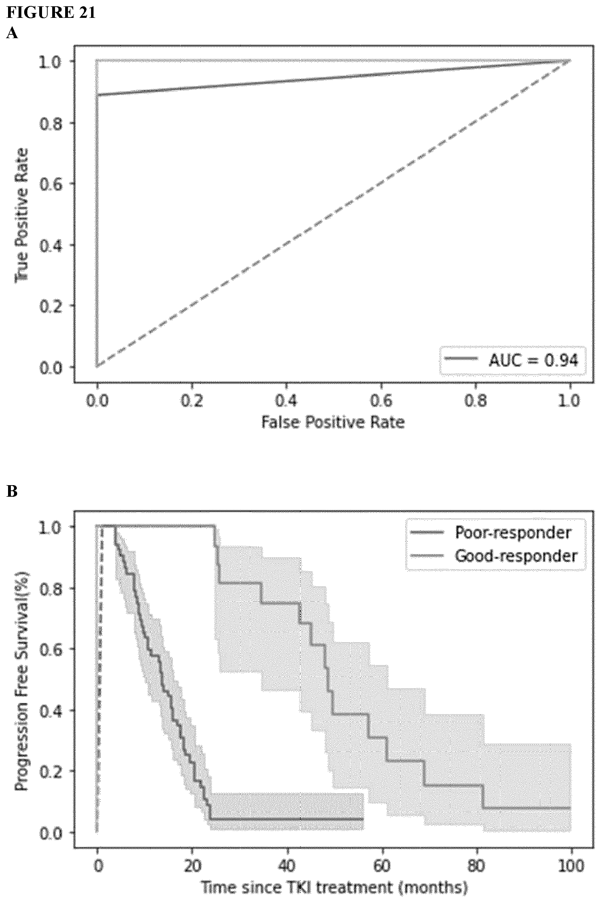

Moreover, GBM models combining blood 15 NNMT related with clinical data demonstrated that the calculated risk score was able to significantly improve the prediction of clinical outcome in patients with EGFR-mutated NSCLC treated with TKI. Our predictive model associated to 2-years overall survival obtained a p value <0.005 in Kaplan-Meier analysis **(****Figure 21B****)** with high accuracy (AUC: 0.94; Accuracy: 0.97; Sensitivity: 1.0; Specificity: 0.88) **(****Figure 21A****).**

The results confirmed a high discriminatory power of blood NNMT related metabolites to distinguish between good and poor responders against TKI therapy in NSCLC. These data indicate that our method using mass spectrometry to measure blood NNMT related metabolites and machine learning models is a promising tool that could be used to support a better management of cancer's patients. Even though these results have been obtained in a small cohort of patients, so it should be validated in a larger number of patients, it should be noted that the models obtained are based on machine learning strategies that allow the improvement of performance with adding new data.

In conclusion, using mass-spectrometry quantification of NNMT related metabolites and machine learning models we can identify which patients present an increase in NNMT levels and therefore a weak response to TKI treatment, resulting in a low survival compared to those patients with low NNMT levels and a better response to TKIs. This method could be used in clinical practice for the rapid and accurate identification of patients with a high risk of progression who may benefit from more aggressive treatment and may improve therapeutic strategies for patients with NSCLC.

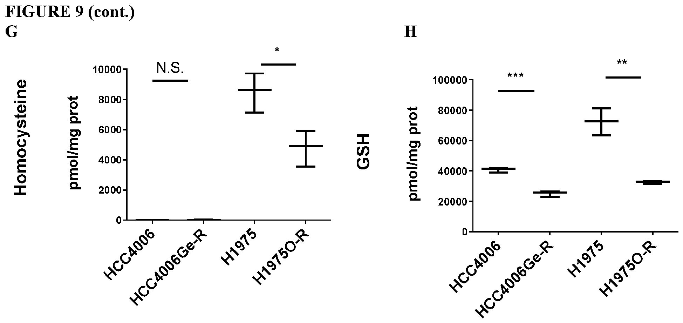

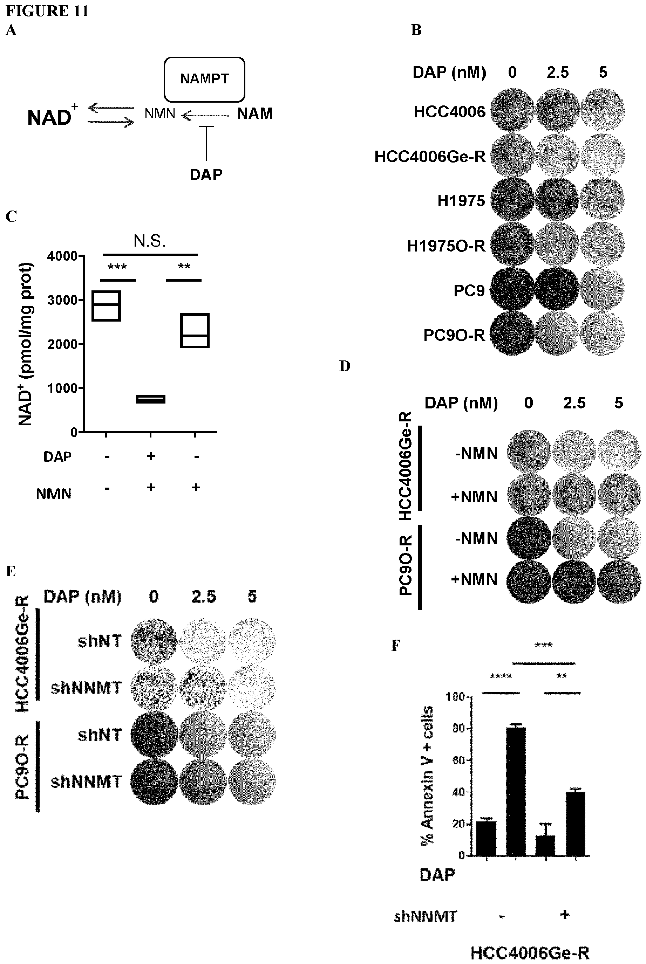

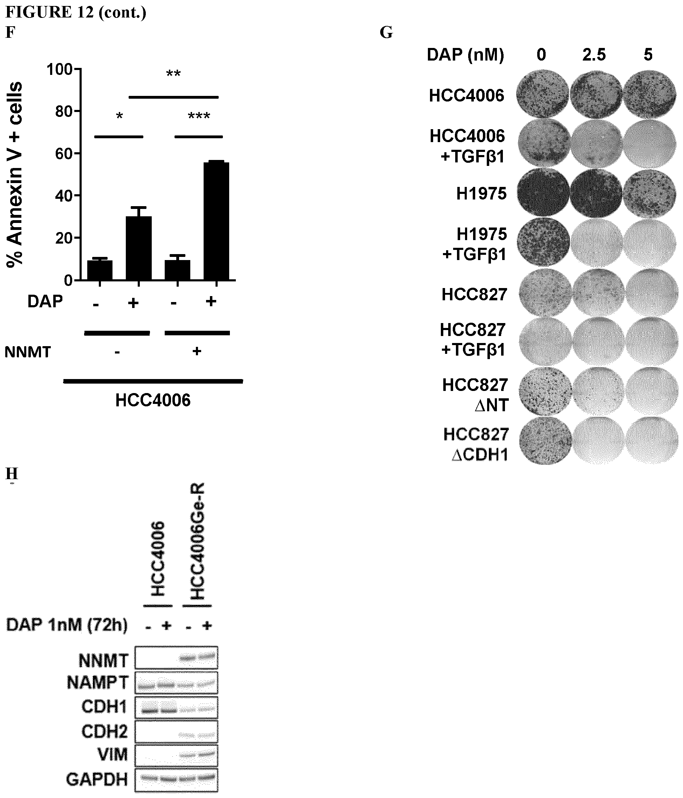

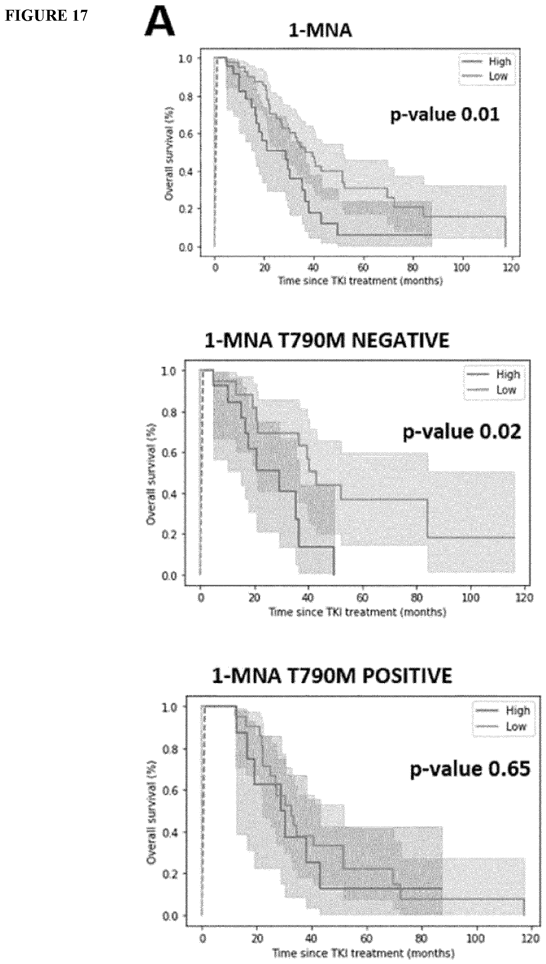

Thus, according to the inventors of the present invention, the main conclusions have been reached:
- NNMT correlates with a mesenchymal phenotype **(****Figure 1****).**
- NNMT correlates with high aggressiveness and survival in NSCLC **(****Figure 2****).**
- NNMT correlates with resistance to cancer therapy **(****Figure 4C & 4D****).**
- NNMT correlates with metabolites from methionine cycle, trans-sulfuration pathway. cystine/cysteine cycle, polyamine pathways and NAD+ salvage cycle, specially,1-MNA **(****Figure 8** **&** **9****).**
- TKI-resistance correlates with metabolites from methionine cycle, trans-sulfuration pathway, cystine/cysteine cycle, polyamine pathways and NAD+ salvage cycle, specially, 1-MNA **(****Figure 8** **&** **9****).**
- NNMT contributes to NAD+ pathway homeostasis **(****Figure 8** **&** **9****).**
- NAMPT inhibition targets a vulnerability specific to cells overexpressing NNMT **(****Figure 11F** **&** **12F**).
- Inhibiting NAMPT seems then a useful approach when NNMT-mediated EMT cells become the predominant population in the tumor **(****Figure 11F****).**
- Identification of patients with high levels of NNMT could serve to predict the response to NAMPT inhibitors **(****Figure 10A****, 10F,** **11G** **&** **12E****).**
- Inhibition of NNMT activity and TKI presents improved results than TKI alone **(****Figure 6E****).**
- Combination of NNMT and NAMPT inhibitors could be reduce aggressiveness and drug resistance, specially, in TKI therapies (Figure 13B).
- 1-MNA is herein presented as a strong biomarker for predicting the response of patients to a treatment with TKIs, or for classifying patients into responder or non-responder patients to a treatment with TKIs. The quantitative measurement of 1-MNA, the by-product of NNMT activity, preferably in liquid biopsies, is correlated with poor prognosis of TKI-treated patients (patients are resistant to the treatment with TKIs) and emerges as a potential biomarker of drug response and metastatic progression, particularly in NSCLC **(****Figure 14A****).**
- Those patients showing a quantitative level of 1-MNA, which, according to the previous paragraph, would be resistant (non-responder) to a treatment with TKIs, could be treated with NNMT inhibitors **(****Figure 11F****,** **13A****,** **13B** **&** **13F****).**
- Like 1-MNA, other metabolites involved in NNMT activity such as S-Adenosyl methionine (SAM), S-adenosyl-L-homocysteine (SAH), Spermine and Spermidine can be good markers for predicting the response of patients to a treatment with TKIs, or for classifying patients into responding or non-responding patients to a treatment with TKIs. The quantitative measurement of these biomarkers, preferably in liquid biopsies, is correlated with poor prognosis of TKI-treated patients (patients are resistant to the treatment with TKIs) and emerges as a potential biomarker of drug response and metastatic progression, particularly in NSCLC **(****Figure 17****).**
- The quantification of other metabolites associated with NNMT activity, such as S-Adenosyl methionine (SAM), 5'-methylthioadenosine (5-MTA), Methionine, Cystine, Nicotinamide, Spermidine, Spermine, Cystathionine, Cysteine, HomoCysteine, Glutamyl-L- cysteine (GluCys), S-adenosyl-L-homocysteine (SAH), Glutathione Oxidized (CSSG), Glutathione (GSH) and Glutathione disulphide (GSSG), can be used to estimate 1-MNA levels **(****Figure 18B****).**
- Machine learning models using quantitative measurement of NNMT related metabolites, preferably in liquid biopsies, is herein presented as a good method for predicting the response of patients to a treatment with TKIs, or for classifying patients into responder or non-responder patients to a treatment with TKIs. Several combinations of 1-MNA, SAM, SAH, 5-MTA, Methionine, Cystine, Nicotinamide, Spermidine, Spermine, Cystathionine, Cysteine, HomoCysteine, GluCys, CSSG, GSH are correlated with poor prognosis of TKI-treated patients (patients are resistant to the treatment with TKIs) and emerges as a potential method of drug response and metastatic progression, particularly in NSCLC. Combination of these metabolites improve the predictive capacity of metabolites individually (Figure 19 & 20). Machine learning models using quantitative measurement of NNMT related metabolites, preferably in liquid biopsies, and clinical data (age, sex, smoking history, tumour stage) is herein presented as a good method for predicting the response of patients to a treatment with TKIs, or for classifying patients into responder or non-responder patients to a treatment with TKIs. Several combinations of 1-MNA, SAM, SAH, 5-MTA, Methionine, Cystine, Nicotinamide, Spermidine, Spermine, Cystathionine, Cysteine, HomoCysteine, GluCys, CSSG, GSH, sex, age, sex, smoking history or tumour stage are correlated with poor prognosis of TKI-treated patients (patients are resistant to the treatment with TKIs) and emerges as a potential method of drug response and metastatic progression, particularly in NSCLC. Combination of these features improve the predictive capacity of each individual features **(****Figure 21****).**

So, the special technical feature which confers unity of invention to the present invention is the use of metabolites or combinations thereof for assessing the activity of NNMT.

The most reliable metabolites and combinations thereof determined in the present invention are summarized in **Table 1, 2** and **3** below:

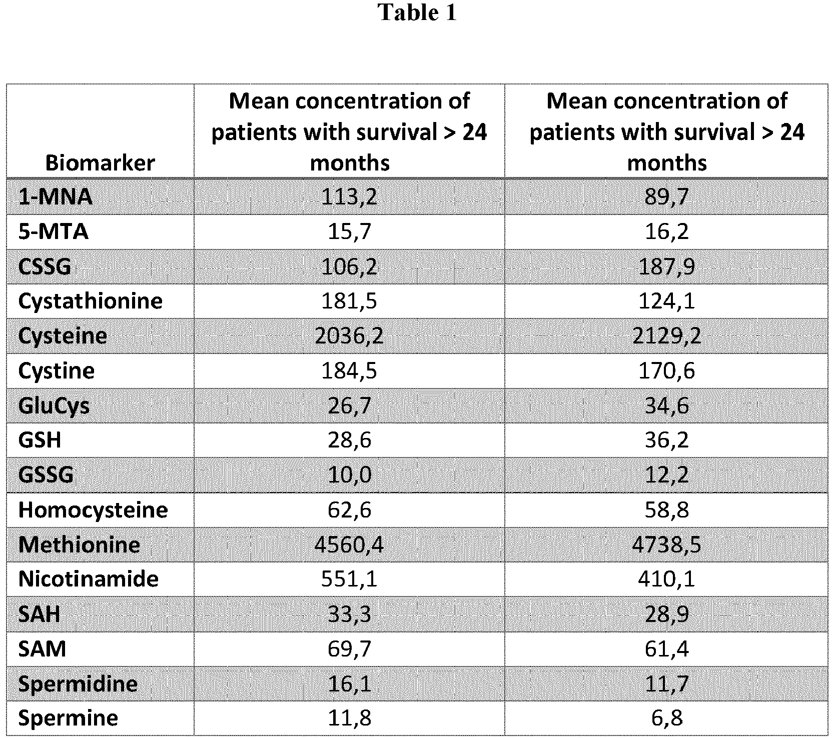

*Levels of NNMT-related metabolites associated to response against TKIs. Mean of serum metabolites UPLC-MS concentration in TKI-treated NSCLC patients with Overall Survival (OS) >24 months or OS* < *24 months.*

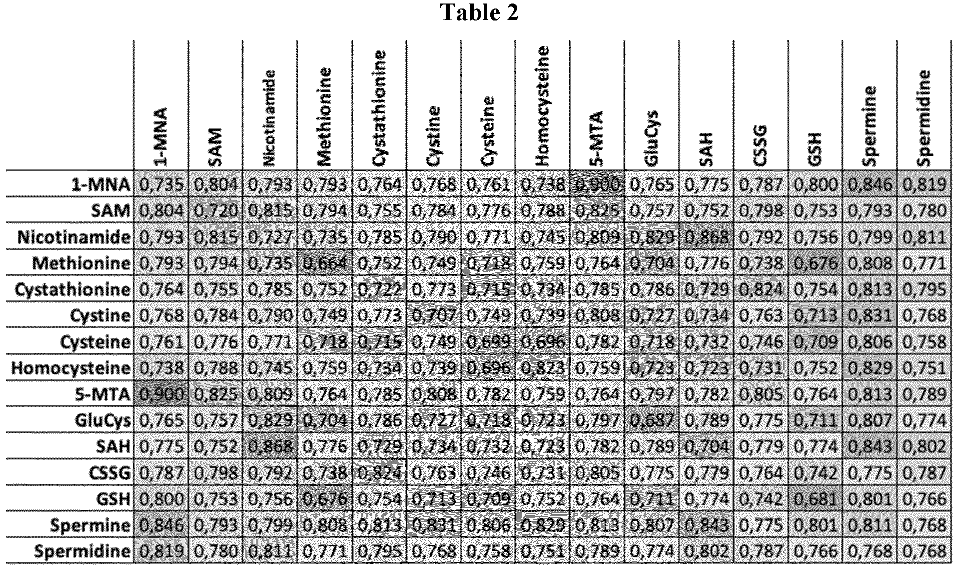

*Performance of 24-months overall survival prediction models using two metabolites from the group NNMT-related metabolites. Area under the ROC curve (AUC) for GBM model using combinations of 2 metabolites from UPLC-MS*/*MS quantification for 1-MNA*, *SAM, Nicotinamide, Methionine, Cystathionine, Cystine, Cysteine, Homocysteine, 5-MTA, GluCys, SAH, CSSG, GSH, Spermine and*/*or Spermidine in blood of TKI-treated NSCLC patients against Overall Survival (OS) at 24 months.*

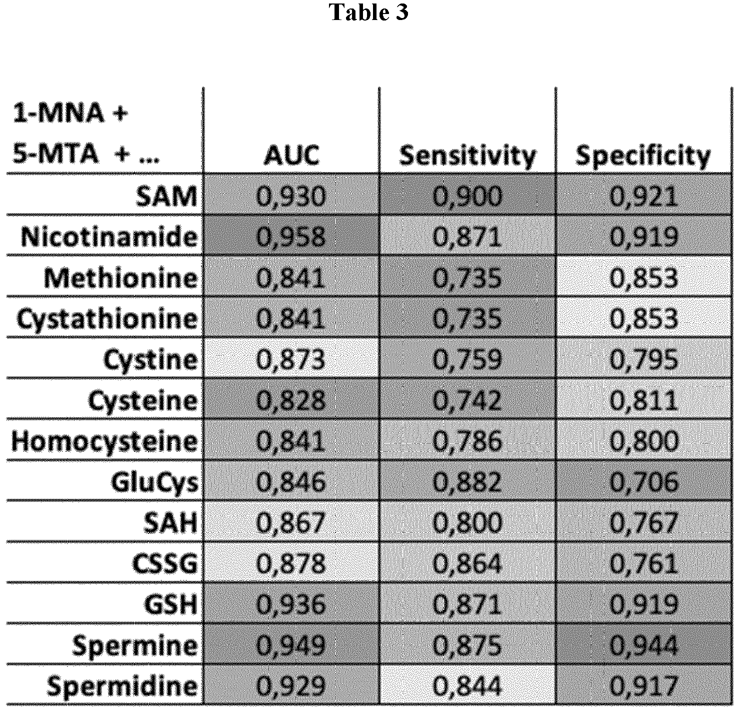

*Performance of 24-months overall survival prediction models of TKIs using 1-MNA*, *5-MTA and other metabolites from the group NNMT-related metabolites. Area under the ROC curve (AUC), sensitivity and specificity for GBM model using combinations of 1-MNA*, *5-MTA and a third metabolite from Nicotinamide, Methionine, Cystathionine, Cystine, Cysteine, Homocysteine, GluCys, SAH, SAM, CSSG, GSH, Spermine and*/*or Spermidine using UPLC-MS*/*MS quantification in blood of TKI-treated NSCLC patients against Overall Survival (OS) at 24 months.*

Consequently, the first embodiment of the present invention refers to an *in vitro* method for assessing the activity of nicotinamide N-methyltransferase (NNMT) in a biological sample (hereinafter *"method of the invention* ") which comprises: a) Determining the concentration level of at least a metabolite selected from the group comprising: 1-MNA, 5-MTA, CSSG, Cystathionine, Cysteine, Cystine, GluCys, GSH, Homocysteine, Methionine, Nicotinamide, SAH, SAM, Spermidine and/or Spermine, or any combination thereof; b) Wherein the identification of a concentration level of 1-MNA, Cystathionine, Cystine, Homocysteine, Nicotinamide, SAH, SAM, Spermidine and/or Spermine higher than a pre-established threshold level is an indication of high NNMT activity; or wherein the identification of a concentration level of 1-MNA, Cystathionine, Cystine, Homocysteine, Nicotinamide, SAH, SAM, Spermidine and/or Spermine lower than a pre-established threshold level is an indication of low NNMT activity; and c) Wherein the identification of a concentration level of 5-MTA, CSSG, Cysteine, GluCys, GSH and/or Methionine lower than a pre-established threshold level is an indication of high NNMT activity; or wherein the identification of a concentration level of 5-MTA, CSSG, Cysteine, GluCys, GSH and/or Methionine higher than a pre-established threshold level is an indication of low NNMT activity.

Kindly note that the activity of a protein to be used as a biomarker does not always depend on, or is not always directly related to, expression levels or protein levels because, for example, there could be mutations or other interactions/collaborations with other activities/pathways that affect the activity, so that if we measure only the expression or protein level, we may not be correctly identifying the activity of the protein.

In a preferred embodiment, the present invention refers to an *in vitro* method for selecting patients who may benefit from a treatment with NNMT inhibitors which comprises assessing the NNMT activity following the method of the invention, wherein the identification of high NNMT activity is an indication that the patients may benefit from a treatment with NNMT inhibitors.

In a preferred embodiment, the present invention refers to an *in vitro* method for the prognosis and/or predicting the response of patients to NNMT inhibitors which comprises assessing the NNMT activity following the method of the invention, wherein the identification of high NNMT activity is an indication of poor prognosis and that the patients may response to a treatment with NNMT inhibitors.

In a preferred embodiment, the present invention refers to an *in vitro* method for predicting whether a patient is resistant to a treatment with tyrosine kinase inhibitors which comprises the identification of high NMMT activity following the method of the invention.

In a preferred embodiment, the present invention refers to an *in vitro* method for screening NNMT inhibitor candidates which comprises: a) Determining the concentration level of at least a metabolite selected from the group comprising: 1-MNA, 5-MTA, CSSG, Cystathionine, Cysteine, Cystine, GluCys, GSH, Homocysteine, Methionine, Nicotinamide, SAH, SAM, Spermidine, and/or Spermine, or any combination thereof, after administering the candidate NNMT inhibitor; a b) wherein the identification of a concentration level of 1-MNA, Cystathionine, Cystine, Homocysteine, Nicotinamide, SAH, SAM, Spermidine and/or Spermine lower than a pre-established threshold level; or wherein the identification of a concentration level of 5-MTA, CSSG, Cysteine, GluCys, GSH and/or Methionine higher than a pre-established threshold level, is an indication of the candidate is a NNMT inhibitor.

In a preferred embodiment, the method of the invention comprises determining the concentration level of at two metabolites selected from the group: 1-MNA, 5-MTA, CSSG, Cystathionine, Cysteine, Cystine, GluCys, GSH, Homocysteine, Methionine, Nicotinamide, SAH, SAM, Spermidine and/or Spermine.

In a preferred embodiment, the method of the invention comprises determining the concentration level of at least the following two metabolites: 1-MNA and 5-MTA, 1-MNA and SAM, and/or 5-MTA and SAM

In a preferred embodiment, the method of the invention comprises determining the concentration level of at two metabolites selected from the group: 1-MNA, 5-MTA, CSSG, Cystathionine, Cysteine, Cystine, GluCys, GSH, Homocysteine, Methionine, Nicotinamide, SAH, Spermidine and/or Spermine; in combination with SAM

In a preferred embodiment, the method of the invention comprises determining the concentration level of at least the following three metabolites: 1-MNA, 5-MTA and SAM

In a preferred embodiment, the method of the invention comprises the patient is suffering from lung cancer, preferably from non-small cell lung cancer, and most preferably from non-small cell lung cancer with EGFR mutation.

In a preferred embodiment, the method of the invention comprises the biological sample obtained from the patients is serum, blood, plasma, sputum, biopsy, urine or cell cultures or cell supernatants.

In a preferred embodiment, the method of the invention is carried by by mass spectrometry-based method.

The second embodiment of the invention refers to the *in vitro* use of at least a metabolite selected from the group comprising: 1-MNA, 5-MTA, CSSG, Cystathionine, Cysteine, Cystine, GluCys, GSH, Homocysteine, Methionine, Nicotinamide, SAH, SAM, SAM, Spermidine and/or Spermine, or any combination thereof, for assessing NNMT activity, for selecting patients which may benefit from a treatment with NNMT inhibitors, for the prognosis and/or predicting the response of patients to NNMT inhibitors, for predicting whether a patient is resistant to a treatment with tyrosine kinase inhibitors and/or for screening NNMT inhibitor candidates.

The third embodiment of the present invention refers to NNMT inhibitors for use in the treatment of patients, wherein the biological sample obtained from the patients is characterized by a concentration level of: a) 1-MNA, Cystathionine, Cystine, Homocysteine, Nicotinamide, SAH, SAM, Spermidine and/or Spermine higher than a pre-established threshold level; or b) 5-MTA, CSSG, Cysteine, GluCys, GSH and/or Methionine lower than a pre-established threshold level.

In a preferred embodiment, the NNMT inhibitor is selected from the list comprising: SAH, Sinefungin, 1-MNA, 1-MQ, 5-amino-1-MQ, 8-Methyl-1-MQ, JBSNF-000088, JBSNF-000265, AK-2, AK-4, MvH45, MS2756, MS2734, GYZ-78, NS1, LL320, AK-12, RS004, HS58A-C2, HS312, 4-Chloropyridine analogs, Yuanhuadine, EL-1, Cyclic peptide, 4-hydroxy-3-methoxybenzaldehyde, other direct or indirect inhibitors and its derivatives.

On the other hand, the present invention also refers to an *in vitro* method for predicting whether a patient is resistant to a treatment with tyrosine kinase inhibitors which comprises determining the level of any of the above metabolites or combinations thereof.

Alternatively, the present invention also refers to a method for detecting the above cited metabolites or combinations thereof in a test sample from a human subject with the objective of detecting NNMT activity.

In a preferred embodiment, the present invention is a computer-implemented invention, wherein a processing unit (hardware) and a software are configured to:
- Receive the concentration level values of any of the above cited metabolites,
- Process the concentration level values received for finding substantial variations or deviations, and
- Provide an output through a terminal display of the variation or deviation of the concentration level.

For the purpose of the present invention the following terms are defined:
- The term "comprising" means including, but it is not limited to, whatever follows the word "comprising". Thus, use of the term "comprising" indicates that the listed elements are required or mandatory, but that other elements are optional and may or may not be present.
- By "consisting of" means including, and it is limited to, whatever follows the phrase "consisting of'. Thus, the phrase "consisting of" indicates that the listed elements are required or mandatory, and that no other elements may be present.
- By "therapeutically effective dose or amount" is intended an amount that, when administered as described herein, brings about a positive therapeutic response in a patient. The exact amount required will vary from subject to subject, depending on the age, and general condition of the subject, the severity of the condition being treated, mode of administration, and the like. An appropriate "effective" amount in any individual case may be determined by one of ordinary skill in the art using routine experimentation, based upon the information provided herein.
- A reference control level is herein mentioned when referring to a "pre-established threshold level". A reference value can be a "threshold value" or a "cut-off" value. Typically, a "threshold value" or "cut-off value" can be determined experimentally, empirically, or theoretically. According to the present invention, the "threshold" value refers the level of metabolite determined as mean, median, or the optimal cut-off value obtained using receiver operator characteristic analysis such as Youden's index, Euclidean index or other recommended indexes.

### Description of the figures

**Figure 1****. NNMT is correlated with a mesenchymal phenotype.** (A) Correlation between *NNMT* expression and genes unique to epithelial or mesenchymal phenotypes in LAD patients (Battle Study). Pearson correlation coefficient and p-value were FDR adjusted. ^{∗∗∗∗} P<0.0001. (B) NNMT expression level positively correlates with gene expressions unique to a mesenchymal phenotype in lung adenoma carcinoma cell lines from CCLE dataset (Broad Institute).
**Figure 2****. Increased NNMT expression correlates with poor prognosis in mutant EGFR NSCLC patients.** NNMT expression analyzed by RT-PCR significantly correlates (p = 0.0454) with poor progression free survival in a cohort of patients with early stage surgically resected lung adenocarcinoma.
**Figure 3****. Increased NNMT expression correlates with tissue fibrosis in Idiopathic pulmonary fibrosis (IPF) patients.** Immunohistochemical analysis of Hematoxylin/eosin (HE), Masson's Trichrome (TRI) and α-SMA and NNMT stains on lung sections from IPF and control patients (C).
**Figure 4****. NNMT is overexpressed in EGFR mutant cells resistant to TKI with a mesenchymal phenotype.** (A) Transcriptomic and proteomic data integration. Protein and mRNA from 1484 single protein are represented as the median ratio between EGFR TKI resistant/TKI sensitive (log2 scale) from 3 different cell line models (HCC4006/Ge-R. HCC827/ERC4. H1975/CLR). X axis shows mRNA levels, Y axis, protein levels. Vertical and horizontal lines represent the threshold used (log2ratio >1 or. -1; >2 or <0.5 in a natural scale. (B) 72-hours cell viability assay with EGFR inhibitors. Data points are average of duplicate wells from three independent assays. Ge-R: gefitinib resistant, O-R: Osimertinib resistant, TGFB1: cells exposed to TGF-b1 to acquire a mesenchymal phenotype, ΔNT: stably express shRNA against non-target (NT), and ΔCDH1: stably express shRNA against E-cadherin (CDH1). Error bars, SD. (C) Increased expression of vimentin (VIM) and decreased expression of E-cadherin (CDH1) in EGFR mutant NSCLC cells grown resistant to EGFR TKIs correlates with NNMT expression. A representative immunoblots from 3 independent experiments. CDH1: E-cadherin, CDH2: N-cadherin, VIM: vimentin, NNMT, GAPDH. (D) NNMT expression starts to increase 85 days after exposure to the increasing concentrations of gefitinib during the generation of HCC4006 cells with acquired resistance to gefitinib. A representative immunoblot from 3 independent experiments (E) Induction of a mesenchymal phenotype by TGFβ-1 exposure (left) or depletion of E-cadherin (ΔCDH1) (center) or by acquired osimertinib resistance (right) promote NNMT expression with increased expression of mesenchymal markers. A representative immunoblots from 3 independent experiments. CDH1: E-cadherin. VIM: vimentin. ACTB: β-actin.
**Figure 5****. NNMT is increased in EGFR mutant tumors resistant to TKI in NSCLC patients.** (A) Clinicopathologic features of the 24 EGFRmut NSCLC patients analyzed by NNMT IHC. (B) Representative immunohistochemical analysis of tumors from NSCLC patients harboring EGFR secondary mutation (T790M⁺) or no T790M (T790M⁻) using anti-NNMT antibody before EGFR TKI therapies (PRE-TKI) and at the time of clinical progression on EGFR TKI therapies (POST-TKI).
**Figure 6****. NNMT is necessary but not sufficient for the maintenance of a mesenchymal phenotype and an EGFR TKI resistance.** (A) Immunofluorescence using antibodies against NNMT (green). E-cadherin (green, CDH1) and vimentin (blue, VIM) in EGFR TKI-resistant EGFR mutant cells stably expressing shRNA targeting non-target (shNT) or shRNA against NNMT (shNNMT) (B) Immunofluorescence using antibodies against NNMT (red). E-cadherin (green. CDH1). vimentin (blue. VIM). and nucleus (gray) in EGFR TKI-sensitive EGFR mutant cells stably expressing V5-tagged control (Flag) or NNMT cDNA. (C) Immunoblots demonstrating that NNMT depletion in EGFR TKI resistant cells with a mesenchymal phenotype (PC9O-R. HCC4006Ge-R. H1975O-R) promotes the expression of an epithelial marker E-cadherin (CDH1) while downregulates the expression of mesenchymal markers N-cadherin (CDH2), and vimentin (VIM). Representative immunoblots from 3 independent experiments are shown. Vinculin (VCL) as a loading control. (D) Immunoblots showing the status of canonical epithelial marker (CDH1: E-cadherin) and mesenchymal markers (CDH2: N-cadherin and VIM: vimentin) in EGFR TKI-sensitive EGFR mutant HCC4006 or H1975 cells stably expressing V5-tagged control (Flag) or NNMT cDNA and PC9 stably expressing mutant (MUT) or wild-type (WT) NNMT cDNA. A representative immunoblots from 3 independent experiments. (E) HCC4006Ge-R and PC9O-R cells stably expressing shRNA targeting NT or NNMT cells were treated with 1 µM of gefitinib and osimertinib. respectively for 72 hours and annexin V assay was performed. Percentages of annexin V positive cells are shown. Average of three independent experiments. Error bars: S.D; One-way ANOVA. N.S. Not significant. ^{∗∗} P<0.01, ^{∗∗∗} P<0.001, N.S. Not significant. (F) HCC4006 cells stably expressing V5-tagged control (Flag) or NNMT cDNA and PC9 stably expressing mutant (MUT) or wild-type (WT) NNMT cDNA were treated with 1 µM of TKI for 72 hours and annexin V assay was performed. Percentage of annexin V positive cells is represented. Average of three independent experiments. Error bars: S.D; One-way ANOVA. N.S. Not significant. ^{∗} P<0.05, ^{∗∗} P<0.01, ^{∗∗∗} P<0.001.
**Figure 7****. NNMT expression decreases the inhibition of cell viabilities by TKI treatment.** (A) 72-hours cell viability assay with EGFR inhibitor in EGFR TKI-R HCC4006Ge-R and PC9O-R cells stably expressing non-target (shNT) or shRNA against NNMT (shNNMT). Data points are average of duplicate wells from three independent assays. Error bars. SD. (B) HCC4006Ge-R and PC9O-R cells stably expressing targeting non-target (shNT) or shRNA against NNMT (shNNMT)were treated with the indicated concentration of gefitinib and osimertinib respectively for 10 days and surviving cells were stained with crystal violet. Image is representative of three independent assays. (C) HCC4006Ge-R. H1975O-R and PC9O-R stably expressing targeting non-target (shNT) or shRNA against NNMT (shNNMT) shRNA targeting NT or NNMT cells were treated with DMSO or 500nM of the indicated TKI for 1 hour. Lysates were subjected to immunoblots with antibodies indicated. Images are representative of three independent experiments. (D) HCC4006 stably expressing control (Flag-V5) or NNMT-V5 expression vector and PC9 cells stably expressing WT or MUT NNMT cDNA were treated with DMSO or 500 nM of the indicated TKI for 1 hour. Lysates were subjected to immunoblot with antibodies indicated. Image is representative of three independent experiments. (E) The indicated cell lines overexpressing NNMT were treated with 5 or 15 nM of TKI for 10 days and surviving cells were stained with crystal violet. Image is representative of three independent assays. (F) 72-hours cell viability assay with osimertinib in HCC4006 stably expressing V5-tagged control (Flag) or NNMT cDNA treated with vehicle or 100 nM osimertinib for 30 days. Data points are average of duplicate wells from three independent assays. Error bars. SD.
**Figure 8****. NAD⁺ metabolism is altered in EGFR mutant cells with a mesenchymal phenotype.** (A) Graphic representation depicting the critical roles of NNMT in the methionine cycle and associated metabolic pathways. UPLC-MS/MS quantification of (B) 1-MNA. (C) NAD⁺. (D) nicotinamide. and (E) SAM/SAH ratio in the supernatants from EGFR mutant NSCLC cells (HCC4006 and H1975) and cells grown resistant to gefitinib (HCC4006Ge-R) or osimertinib (H1975O-R). Average of three independent experiments. Error bars: Min to max; T-test. ^{∗} P<0.05, ^{∗∗} P<0.01, ^{∗∗∗} P<0.001, ^{∗∗∗∗} P<0.0001.
**Figure 9****. Methionine. polyamine. trans-sulphuration metabolism is altered in EGFR mutant cells with a mesenchymal phenotype.** (A) Graphic representation depicting the critical roles of NNMT in the methionine cycle and associated metabolic pathways. UPLC-MS/MS quantification of (A) methionine, (B) homocysteine, (C) 5-MTA, (D) cystathionine, (E) spermidine, (F) cysteine, (G) spermine and (H) GSH in in the supernatants from EGFR mutant NSCLC cells (HCC4006 and H1975) and cells grown resistant to gefitinib (HCC4006Ge-R) or osimertinib (H1975O-R). Average of three independent experiments. Error bars: Min to max; T-test. N.S. Not significant. ^{∗} P<0.05, ^{∗∗} P<0.01, ^{∗∗∗} P<0.001.
**Figure 10****. Metabolite biomarkers of NNMT activity.** UPLC-MS/MS quantification of 1-MNA. NAD⁺. nicotinamide and SAM/SAH ratio in supernatants of (A) HCC4006Ge-R cells stably expressing non-target (shNT) or shRNA against NNMT (shNNMT), and (B) HCC4006 stably expressing V5-tagged control (Flag) or NNMT cDNA. Average of three independent experiments. Error bars: Min to max; T-test. N.S. Not significant, ^{∗} P<0.05, ^{∗∗} P<0.01.

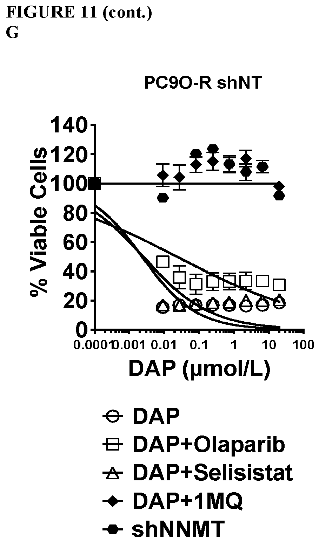

**Figure 11****. EGFR TKI resistance by NNMT overexpression can be reverted by Daporinad via NAD+ depletion.** (A) Graphical representation of NAMPT-mediated NAD⁺ synthesis pathway and inhibition by daporinad (DAP). (B) HCC4006/HCC4006Ge-R. H1975/H1975OR and PC9/PC9O-R cells were treated with the indicated concentration of DAP for 10 days and surviving cells were stained with crystal violet. The displayed image is representative of three independent assays. (C) UPLC-MS/MS quantification of NAD⁺ in HCC4006O-R cells after 24-hours DAP (1 µM) and DAP with nicotinamide mononucleotide (NMN. 100 µM) treatment combination. Error bars: Min to max; One-way ANOVA. N.S. Not significant. ^{∗∗} P<0.01. ^{∗∗∗} P<0.001. (D) HCC4006Ge-R. and PC9O-R cells were treated with the indicated concentration of DAP and DAP with NMN (100 µM) for 10 days and surviving cells were stained with crystal violet. Image is representative of three independent assays. (E) NNMT depletion diminishes DAP sensitivity in HCC4006Ge-R and PC9O-R cells. Following the 10-day DAP treatment. surviving cells were stained with crystal violet. Image is representative of three independent assays. (F) Percentages of annexin V positive cells after 72-hour DAP treatment in TKI-resistant HCC4006Ge-R cells stably expressing shRNA targeting non-target (shNT) or shRNA against NNMT (shNNMT). Average of three independent experiments. Error bars: S.D; One-way ANOVA. ^{∗∗} P<0.01. ^{∗∗∗} P<0.001. ^{∗∗∗∗} P<0.0001. (G) PC9O-R shNT cells were subject to colorimetric *in vitro* proliferation assay for 72 hours with DAP, olaparib, selisistat or its combination and compared to PC9O-R shNNMT cells treated with DAP. Data points are average of duplicate wells from three independent assays. Error bars, SD.

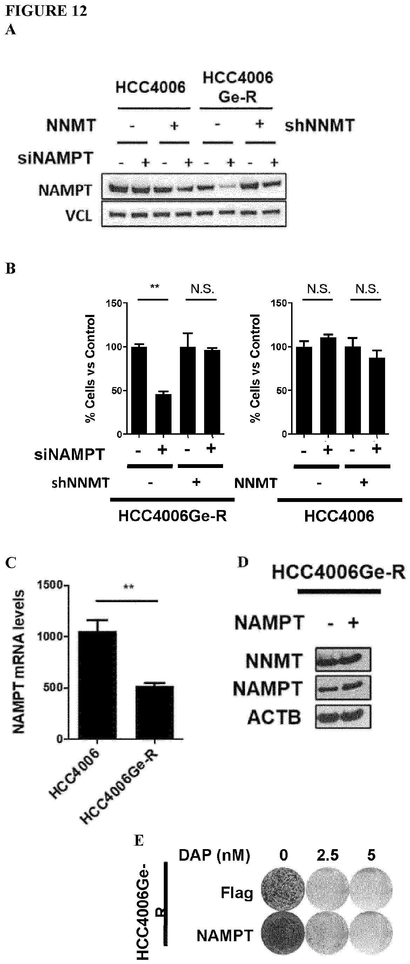

**Figure 12****. Combination of NNMT and NAMPT inhibition improve EGFR TKI sensitivity.** (A) HCC4006 stably expressing V5-tagged control (Flag) or NNMT cDNA and HCC4006Ge-R cells stably expressing shRNA targeting NT or NNMT were transfected with siRNA targeting NAMPT or Control. NAMPT. VCL. A representative immunoblots from three independent experiments. (B) 72 hours post-transfection with siRNA targeting NAMPT or Control. % Surviving cells were assessed using HCC4006Ge-R cells stably expressing shRNA targeting NT or NNMT (left) and HCC4006 cells stably expressing V5-tagged control (Flag) or NNMT cDNA (right). Error bars: S.D; One-way ANOVA. N.S. Not significant. ^{∗∗} P<0.01. (C) The quantification of NAMPT mRNA levels in HCC4006 and HCC4006Ge-R cells using Taqman RT-PCR. Error bars: S.D; T-test. ^{∗∗} P<0.01. (D) HCC4006Ge-R cells stably expressing NAMPT maintain NNMT protein levels. NNMT. NAMPT. ACTB: β-actin. A representative immunoblots from three independent experiments. (E) HCC4006Ge-R cells ectopically overexpressing NAMPT cDNA were treated with the indicated concentration of DAP for 10 days and surviving cells were stained with crystal violet. Image is representative of three independent assays. (F) Annexin V assay was performed after 72-hour DAP treatment in HCC4006 cells stably expressing control (Flag-V5) or NNMT-V5 expression vector. Percentage of annexin V positive cells is represented. Average of three independent experiments. Error bars: S.D; One-way ANOVA. ^{∗} P<0.05. ^{∗∗} P<0.01. ^{∗∗∗} P<0.001. (G) The indicated cell lines were treated with DAP for 10 days. Surviving cells were stained with crystal violet. Image is representative of three independent assays. (H) HCC4006 and HCC4006Ge-R cells were treated with DAP 1nM for 72h without changes in their epithelial or mesenchymal phenotype. E-cadherin (CDH1). N-cadherin (CDH2). CD44. vimentin (VIM). NNMT. NAMPT. GADPH. A representative immunoblots from 3 independent experiments.
**Figure 13****. The inhibition of NNMT activity promotes mesenchymal to epithelial transition to ameliorate EGFR TKI sensitivity.** (A) UPLC-MS/MS quantification of 1-MNA in H1975O-R and PC9O-R cells treated with 1-MQ (100 µM) for 30 days. Error bars: Min to max; T-test. ^{∗∗∗∗} P<0.0001. (B) Annexin V assay was performed in HCC4006Ge-R shNT cells after 72-hour treatment with DAP (5 nM) and DAP and 1-MQ (100 µM) combination. Percentage of annexin V positive cells is represented. Average of three independent experiments. Error bars: S.D; One-way ANOVA. N.S. Not significant. ^{∗∗∗∗} P<0.0001. (C) % NAD⁺ levels in HCC4006Ge-R cells treated for 72-hours with increasing concentration of DAP with and without 1-MQ (100 µM) compared to vehicle control. Average of three independent experiments. Error bars: S.D; One-way ANOVA. N.S. Not significant. ^{∗} P<0.05. ^{∗∗} P<0.01. ^{∗∗∗} P<0.001. ^{∗∗∗∗} P<0.0001. (D) Immunofluorescence assays of NNMT. E-cadherin and vimentin in EGFR TKI-resistant EGFR mutant cells treated with 1-MQ (100 µM) for 30 days. A representative image from three independent experiments. Red, NNMT; green, E-cadherin; blue, vimentin; grey, nucleus. (E) The indicated cell lines were treated with indicated concentrations of osimertinib for 10 days. Surviving cells were stained with crystal violet. Image is representative of three independent assays. (F) H1975O-R and PC9O-R control and 1-month 1-MQ (100 µM) treated cells were subject to colorimetric *in vitro* proliferation assay for 72 hours with osimertinib. Data points are average of duplicate wells from three independent assays. Error bars. SD. (G) PC9 stably expressing WT or MUT NNMT protein were seeded at low density. treated with vehicle. osimertinib (100 nM) or osimertinib with 1-MQ (100 µM) and grown until confluency. Percent of confluent wells was assessed every week. Data points are average of three independent assays.
**Figure 14****. Serum 1-MNA association with prognostic.** Correlation between serum level of NNMT metabolite. 1-MNA. and progression free survival (PFS) or Overall survival (OS) in a cohort of 69 EGFR mutant NSCLC patients. (A) PFS from all the patients (left), from patients with detectable T790M (middle), and from the patient without detectable T790M mutation upon EGFR TKI treatment (right). (B) OS from all the patients (left), from patients with detectable T790M (middle), and from the patient without detectable T790M mutation upon EGFR TKI treatment (right). 1-MNAhigh/low stratified according above or below the median value respectively. Censored data are indicated by tick marks. Data from patients who had not died at the time of the analysis were censored on the basis of the last recorded date on which the patient was known to be alive. (C) Cox regression analysis of PFS. (D) Cox regression analysis of OS.
**Figure 15****. Clinicopathologic features of the 69 EGFR mutant NSCLC patients in the study.** Clinicopathologic features of the 69 EGFR mutant NSCLC patients in the study.
**Figure 16****. Serum metabolites levels associated with overall survival.** (A) UPLC-MS/MS quantification of NNMT related metabolites in blood of 69 EGFR mutant NSCLC patients. (B) Mean of each subgroup and p-value for Log-Rank test for correlation between serum level of NNMT metabolites and overall survival (OS) in a cohort of 69 EGFR mutant NSCLC patients from all the patients (left). from patients with detectable T790M (middle), and from the patient without detectable T790M mutation upon EGFR TKI treatment (right). High and low groups were stratified according above or below the mean value respectively for each metabolite. Censored data are indicated by tick marks. Data from patients who had not died at the time of the analysis were censored on the basis of the last recorded date on which the patient was known to be alive.
**Figure 17****. Serum metabolites associated with prognostic.** Correlation between serum level of (A) 1-MNA, (B) SAH, (C) SAM, (D) Spermine and (E) Spermidine metabolite and overall survival (OS) in a cohort of 69 EGFR mutant NSCLC patients from all the patients (left), from patients with detectable T790M (middle) and from the patient without detectable T790M mutation upon EGFR TKI treatment (right). High and low groups were stratified according above or below the mean value respectively for each metabolite. Censored data are indicated by tick marks. Data from patients who had not died at the time of the analysis were censored on the basis of the last recorded date on which the patient was known to be alive.
**Figure 18****. Interrelation between metabolites related to NNMT activity.** (A) Spearman correlation to check pairs correlation between metabolites. (B) EGFR status detailed. (B) Receiver-operating characteristics (ROC) curve and area under the ROC curve (AUC) for 1-MNAHigh/Low classification using UPLC-MS/MS quantification of Nicotinamide, Methionine, Cystathionine, Cystine, Cysteine, Homocysteine, 5-MTA, GluCys, SAH, SAM, CSSG, GSH, Spermine and Spermidine in blood.
**Figure 19****. Performance of GBM prediction models by NNMT-related metabolites and 24-months overall survival.** (A) Receiver-operating characteristics (ROC) curve and area under the ROC curve (AUC) for GBM model using UPLC-MS/MS quantification of Nicotinamide, Methionine, Cystathionine, Cystine, Cysteine, Homocysteine, 5-MTA, GluCys, SAH, SAM, CSSG, GSH, Spermine and Spermidine in blood and OS at 24 months. (B) Kaplan-Meier survival curves based for GBM model prediction. Good and Poor responders were stratified according GBM model for OS at 24 months. (C) Importance score of variables for the model.
**Figure 20****. Performance of GBM prediction models by NNMT-related metabolites and 24-months overall survival.** Receiver-operating characteristics (ROC) curve and area under the ROC curve (AUC) for GBM model using combinations from 2 to 15 UPLC-MS/MS quantification for Nicotinamide, Methionine, Cystathionine, Cystine, Cysteine, Homocysteine, 5-MTA, GluCys, SAH, SAM, CSSG, GSH, Spermine and Spermidine in blood and OS at 24 months.
**Figure 21****. Performance of GBM prediction models by NNMT-related metabolites, age (years), sex, stage and smoking status for 24-months overall survival.** (A) Receiver-operating characteristics (ROC) curve and area under the ROC curve (AUC) for GBM model using UPLC-MS/MS quantification of Nicotinamide, Methionine, Cystathionine, Cystine, Cysteine, Homocysteine, 5-MTA, GluCys, SAH, SAM, CSSG, GSH, Spermine and Spermidine in blood, clinical features and OS at 24 months. (B) Kaplan-Meier survival curves based for GBM model prediction. Good and Poor responders were stratified according GBM model for OS at 24 months.

### Detailed description of the invention

The present invention is illustrated by means of the examples below without the intention of limiting its scope of protection.

### Example 1. Material and methods

### Example 1. 1. NSCLC resistant cell lines

HCC4006, HCC827, PC9 AND H1975 EGFR mutant cell lines were exposed to increasing concentrations of EGFR TKIs over 6 months in a manner similar to previously reported [Lynch, T.J., et al., Activating mutations in the epidermal growth factor receptor underlying responsiveness of non-small-cell lung cancer to gefitinib. N Engl J Med, 2004. 350(21): p. 2129-39]. For EGFR TKI-resistant HCC827 cells, clones were used as described previously [Lynch, T.J., et al., Activating mutations in the epidermal growth factor receptor underlying responsiveness of non-small-cell lung cancer to gefitinib. N Engl J Med, 2004. 350(21): p. 2129-39]. All resistant cells are able to proliferate normally in the presence of 10 µmol/L EGFR TKIs. Upon confirming resistance, cells were cultured without drugs and their resistance to TKI was examined periodically.

### Example 1. 2. Western blot analysis

NSCLC cell lines were lysed for 1 hour in Cell Extraction Buffer (Biosource, Camarillo, CA) supplemented with protease and phosphatase inhibitors and precleared by centrifugation before protein quantification using the BCA Protein Assay kit (Pierce, Rockford, IL). Proteins (20 µg) resolved by SDS-PAGE were electrophoretically transferred to polyvinylidene difluoride membrane and Western blots were carried out using standard techniques.

Cells were rinsed twice with cold PBS and proteins were extracted in ice-cold lysis buffer (Cell Signaling, Danvers, MA, USA) containing a protease and phosphatase inhibitors cocktail (Sigma-Aldrich, St. Louis, MO, USA), precleared by centrifugation before protein quantification using the BCA Protein Assay kit (Pierce, Rockford, IL). Proteins (20 µg) resolved by SDS-PAGE were electrophoretically transferred to polyvinylidene difluoride membrane using Bio-rad systems (Bio-rad, Richmond, CA, USA). Blots were incubated overnight at 4 °C with primary antibodies. Blots were incubated for 1 h at room temperature with the appropriate peroxidase-conjugated secondary antibody HRP-linked antibody (Cell Signaling, Danvers, MA, USA). Blots were visualized using a chemiluminescence detection kit ECL Western blotting substrate (Fisher Scientific, Madrid, Spain). Signals were captured by the ChemiDoc^{™} XRS + Imaging System (Bio-rad, Richmond, CA, USA).

### Example 1. 3. Cellular viability assays

Live cells were counted using Countess II (Thermo Fisher Scientific) and 5×10³ cells per well were seeded in 96-well plates to compare cell growth kinetics using Sulforhodamine B assay as described by the NCI. The results were analyzed and graphed using Prism 8 (GraphPad Software). For the crystal violet cell viability assay, cells were seeded at low density (5×10³ cells per well) in 6-well plates and left to adhere for 24 hours. Then media was replaced with vehicle, or the appropriate drug (DAP or TKI) and it was renewed every 3 days until approximately 70% confluency was reached. Supernatant was replaced with fixing solution and cells were stained with crystal violet 0.4% (v/v). Plates were then washed in running water, allowed to dry and scanned for imaging.

### Example 1. 4. Cell death studies with annexin-V staining

Adherent cells were trypsinized and pooled with the supernatant. Apoptosis was assessed using an Annexin V-FITC conjugate (ThermoFisher Scientific) and Propidium Iodide (ThermoFisher Scientific) staining according to the manufacturer's instructions. After incubation, samples were placed on ice and run using a BD LSRFortessa and data was analyzed with FlowJo software. Appropriate unstained and single-stained samples were used for controls and compensation.

### Example 1. 5. Cell death analysis by fluorescence automatized microscopy

INCell Analyzer 2000 (Ge Healthcare Life Sciences) was used to quantify cell death systematically. Briefly, 5×10³ cells were seeded in black-wall 96-well plates and left 24 hours to attach. The appropriate siRNA was added to the wells and 72 hours after treatment, cell death was evaluated in situ with annexin V, propidium iodide and Hoechst 33342 staining. A 20x/0.45, Plan Fluor ELWD objective was used and INCell Investigator Image Analysis 1000 Workstation Software was used to analyze the acquired images.

### Example 1.6. siRNA transfection

siRNA (pooled oligos) was transfected into the target cells using TransIT-siQUEST reagents (Mirus) according to manufacturer's instructions. The catalog numbers for the siRNAs used are as follows:

| siRNA | Catalog Number |
|---|---|
| Control siRNA-A | Santa Cruz Biotechnology sc-37007 |
| NNMT siRNA | Santa Cruz Biotechnology sc-61213 |
| NAMPT siRNA | Santa Cruz Biotechnology sc-45843 |
| NPRT1 siRNA | Santa Cruz Biotechnology sc-77596 |

### Example 1. 7. cDNA/shRNA constructs and lentiviral infection

pLX304 was a gift from David Root (Addgene plasmid # 25890; http://n2t.net/addgene:25890; RRID: Addgene_25890) (1). cDNA for NNMT (HsCD00442343) and NAMPT (HsCD00442891) were cloned to pLX304 vector and obtained from DNASU, Arizona State University Biodesign Institute. cDNA for WT and catalytically inactive NNMT (Y20A) mutant were cloned in pLVX-IRES-ZsGreen or pLVX-IRES-ZsTomato respectivelly.

shRNA lentiviral vector construct designed by the RNAi Consortium was used as described previously [Lynch, T.J., et al., Activating mutations in the epidermal growth factor receptor underlying responsiveness of non-small-cell lung cancer to gefitinib. N Engl J Med, 2004. 350(21): p. 2129-39]. shRNA knockdown and viral transduction were performed as reported previously [Lynch, T.J., et al., Activating mutations in the epidermal growth factor receptor underlying responsiveness of non-small-cell lung cancer to gefitinib. N Engl J Med, 2004. 350(21): p. 2129-39]. Using TransIT-LT1 transfection reagent (Mirus), the 293LTV cell line (Cell Biolabs) was transfected with pLKO.1 constructs and packaging plasmids, pCMV-ΔR8.2dvpr and pCMV-VSV-G. Lentiviral supernatants were collected, and quality of viral supernatant was routinely tested with Lenti-X Go Stix (Clontech). The supernatants were applied to target cells. Following puromycin selection, cell viability assays were performed 7 days post-infection.

| **Target** | **RNAi Consortium Number** | **Sequence** | **SEQ ID NO** |
|---|---|---|---|
| Non-Target | N/A | 5'-GCGCGATAGCGCTAATAATTT-3' | SEQ ID NO: 1 |
| CDH1 | TRCN0000039664 | 5'-AATGCCATCGTTGTTCACTGG-3' | SEQ ID NO: 2 |
| NNMT | TRCN0000035227 | 5'-ACCCTCGGGATTACCTAGAAA- 3' | SEQ ID NO: 3 |

### Example 1. 8. RNA/DNA extraction

RNA from cell lines and fresh frozen tumor or adjacent normal lung tissues was extracted and purified using trizol (TRI Reagent^{®}, Sigma) per manufacturer instructions. DNA was extracted with DNeasy blood & tissue kit (Qiagen). Samples were quantified using NanoDrop ND-200 Spectrophotometer.

### Example 1. 9. Analysis of Microarray Gene Expression Data

Gene expression profiles of indicated experimental conditions were generated using the Human Genome U133A 2.0 microarray (Affymetrix) (Genomics Core Facility, University of Valencia). Affymetrix GeneChipScanner was used for the export of summarized probe intensities. Next, the expression data was annotated and collapsed into Genepattern. Differential expression analysis using the t-test statistic and the false discovery rate (FDR) adjustment was carried out to find differentially expressed transcripts (http://www.broadinstitute.org/cancer/software/genepattern).

GSEA (http://www.broadinstitute.org/gsea) was used to determine the gene set enrichment of experimental signatures related with EMT and E-cadherin knockdown obtained from the MsigDB (http://www.broadinstitute.org/gsea/msigdb). GSEA estimates whether the members of a given gene signature are found at the top or bottom of a list of genes ranked by signal-to-noise ratio, indicating they are associated with a specific phenotype, rather than being distributed uniformly or randomly across the gene list. An enrichment score (ES) is calculated to quantify the degree to which a gene signature is overrepresented at the top or bottom of the entire ranked list. GSEA normalizes the ES for each gene set to account for the variation in set sizes, yielding a normalized enrichment score (NES) and a false discovery rate (FDR). The FDR gives an estimate of the probability that a set with a given NES represents a false positive finding; it is computed by comparing the tails of the observed and empirical phenotype-based permutated null distributions for the NES.

### Example 1. 10. Proteomic analysis

SWATH LC-MS/MS analysis was used to study the proteomic content of individual samples. Briefly, samples were run in acrylamide gels and gel lanes slices and digested with trypsin as described previously [Shevchenko A, Jensen ON, Podtelejnikov AV, Sagliocco F, Wilm M, Vorm O, Mortensen P, Boucherie H, Mann M. Linking genome and proteome by mass spectrometry: large-scale identification of yeast proteins from two dimensional gels. Proceedings of the National Academy of Sciences of the United States of America 1996; 93:14440-14445]. 5 µl of each sample were loaded onto a trap column (NanoLC Column, 3µ C18-CL, 75um×15cm; Eksigen) and desalted with 0.1% TFA at 3µl/min during 5 min. The peptides were loaded onto an analytical column (LC Column, 3 µ C18-CL, 75µm×12cm, Nikkyo) equilibrated in 5% acetonitrile 0.1% FA. Peptide elution was carried out with a linear gradient of 5 to 35% B in 90 min (A: 0.1% FA; B: ACN, 0.1% FA) for at a flow rate of 300nl/min. Peptides were analyzed in a mass spectrometer nanoESI qQTOF (5600 TripleTOF, ABSCIEX).

The tripleTOF was operated in information-dependent acquisition mode, in which a 250-ms TOF MS scan from 350-1250 m/z, was performed, followed by 150-ms product ion scans from 350-1500 m/z on the 25 most intense 2-5 charged ions. The rolling collision energies equations were set for all ions as for 2+ ions. ProteinPilot default parameters were used to generate peak list. The Paragon algorithm of ProteinPilot was used to search SwissProt protein database.

### Example 1.11. NAD⁺/NADH levels quantification

NAD+/NADH-Glo Assay kit (Promega) was used per manufacturer advice as a faster method to quantify NAD+/NADH than UPLC-MS/MS. Briefly, 3.5×10³ cells were seeded in 96-well plates and left 24 hours to adhere before DAP addition. After 24-hour of drug incubation NAD⁺/NADH detection reagent was added. One-hour post incubation supernatant was transferred to a white-well plate to evaluate luminescence in an Infinite M200pro (Tecan).

### Example 1.12. UPLC-MS targeted analysis

UPLC separation was performed in an Acquity UPLC system (Waters, UK) equipped with an Acquity UPLC BEH C18 (1.7 µm, 2.1 × 100 mm; Waters) column. The temperatures of the column and the autosampler were set at 40°C and 4°C, respectively. The sample injection volume was 4 µl. Eluents consisted in 0.5% formic acid and 0.3% heptafluorobutyric acid in water (eluent A) and acetonitrile (eluent B). The flow rate was set at 0.3 ml/min. A 9-min elution gradient was performed as follows: initial eluent composition was set at 97% A and 3% B, which was linearly changed to 90% A and 10% B in 2 min; then the proportion of B was increased to 30% in the next 3 min, followed by a further increase to 100% B reached at min 6 and kept for 1.5 min. Finally, the initial conditions were recovered and maintained for 2 min for column conditioning.

The MS analysis was performed using a Waters Xevo TQ-S mass spectrometer (Waters) equipped with an ESI source working in positive-ion mode multiple reaction monitoring (MRM) mode. A capillary voltage of 2 kV, a source temperature of 120°C and a desolvation temperature of 380°C were used. Desolvation and the cone gas flow were set as 800 liters/h and 150 liters/h, respectively, and the collision gas was 0.25 ml/min. Transitions, cone voltages, and collision energies were automatically tuned for each metabolite using the Quanoptimizer software (Waters). The data station operating software used was MassLynx 4.1 (Waters). MS conditions (including quantification and qualification transitions, cone voltage, collision energy, retention time and internal standard used for normalization purposes) for each metabolite and internal standard are detailed in the following table:

| **Compound** | **Parent Ion** | **Daughter Ion** | **Cone (V)** | **Collision (eV)** | **RT (min)** | **IS** |
|---|---|---|---|---|---|---|
| **Nicotinamide** | **123.1** | **80.1** | **40** | **20** | **2.05** | Theanine |
| | | 78.0 | 40 | 20 | | |
| **1-M-** | **137.1** | **94.0** | **60** | **15** | **2.00** | 1MNAD3 |
| **Nicotinamide** | | 92.0 | 60 | 15 | | |
| **Methionine** | **150.1** | **104.1** | **20** | **10** | **3.76** | MetD3 |
| | | 133.0 | 20 | 10 | | |
| **Cystathione** | **223.1** | **88.0** | **20** | **20** | **2.61** | CystineD6 |
| | | 134.0 | 20 | 15 | | |
| **Cystine** | **241.0** | **152.0** | **40** | **10** | **2.55** | CystineD6 |
| | | 120.0 | 40 | 20 | | |
| **Cysteine (NEM)** | **247.1** | **158.0** | **20** | **20** | **4.00** | PheD5 |
| | | 201.0 | 20 | 10 | | |
| **Homocysteine** | **261.0** | **55.8** | **20** | **15** | **4.64** | PheD5 |
| **(NEM)** | | 215.0 | 20 | 10 | | |
| **GluCys (NEM)** | **376.1** | **247.1** | **20** | **15** | 4.48 | Theanine |
| | | 84.3 | 20 | 40 | | |
| **SAH** | **385.1** | **136.0** | **20** | **20** | **4.52** | PheD5 |
| | | 250.0 | 20 | 10 | | |
| **SAM** | **399.1** | **250.0** | **20** | **10** | **5.11** | PheD5 |
| | | 136.0 | 20 | 30 | | |
| **CSSG** | **427.1** | **298.1** | **40** | **15** | **3.52** | Theanine |
| | | 177.1 | 40 | 20 | | |
| **GSH (NEM)** | **433.1** | **304.1** | **40** | **10** | **4.36** | PheD5 |
| | | 201.0 | 40 | 15 | | |
| **GSSG** | **613.2** | **355.0** | **60** | **20** | **3.95** | Theanine |
| | | 484.1 | 60 | 15 | | |
| **NAD** | **664.0** | **136.0** | **40** | **30** | **0.93** | Aminobenzamide |
| | | 524.0 | 40 | 15 | | |

Calibration curves were generated by plotting the peak area ratio of the respective compound to the corresponding internal standard versus the nominal concentration. The line of best fit was determined by linear-weighted (1/×) least-squares regression. The linearity acceptance criterion for the correlation coefficient was 0.99 or better. Each back calculated standard concentration should be within ±15% deviation from the nominal value, except for the LLOQ, for which the maximum acceptable deviation was ±20%. Standards calibration curves, with concentrations in the 0.62-20,000 nM range, were prepared by serial half dilutions. IS concentrations were kept constant at all the calibration points at 3000nM for 3Aminobenzamide and CystineD6, 300 nM for MetD3 and 5MTA, 200 nM for MNAD3 and 100nM for theanine and PheD5.

### Example 1.13. Sample preparation

Serum samples. First, 100 µl of serum sample was allowed to thaw on ice and then 25 µl of 40 mM N-ethylmaleimide (NEM) were added to derivatize free thiols. Protein precipitation was performed by adding 3 volumes of cold methanol. After vortexing samples were incubated at -20 °C for 30 min. To remove protein precipitate, samples were centrifuged twice at 10,000 g for 10 min at 4°C, and supernatants were transferred to clean tubes. A 400 µl aliquot was evaporated to dryness and resuspended in 80 µl of eluent A containing internal standards. Another aliquot was diluted 1/20 into eluent A containing internal standards.

Cultured cell samples. For 6-well plates. Media was removed and the cells washed twice with cold PBS. Cells were scraped using 1000 µL of 8mM NEM in water. Cells were lysed by using 3 freeze-thaw cycles. Then, a 250 µl was extracted using 750 µl of cold methanol. After vortexing samples were incubated at -20 °C for 30 min. To remove protein precipitate, samples were centrifuged twice at 10,000 g for 10 min at 4°C, and supernatants were transferred to clean tubes. An 800 µl aliquot was evaporated to dryness and resuspended in 80 µl of eluent A containing internal standards. Another aliquot was diluted 1/20 into eluent A containing internal standards.

Cell culture spent media. First, 20 µl of spent media was allowed to thaw on ice and then 5 µl of 40 mM NEM were added. Protein precipitation was performed by adding 3 volumes of cold methanol. After vortexing samples were incubated at -20 °C for 30 min. To remove protein precipitate, samples were centrifuged twice at 10,000 g for 10 min at 4°C, and supernatants were transferred to clean tubes. An aliquot was diluted 1/20 into eluent A containing internal standards.

### Example 1.14. Histology and immunohistochemistry

All immunohistochemistry procedures were performed in the Department of Pathology at Hospital Quirónsalud (Valencia, Spain). In murine studies, tumors were fixed in 4% formalin overnight at room temperature. The specimen was then embedded in paraffin, from which 5-µm sections were generated. Tumors were sectioned on slides with 5 µm thickness. Then paraffin sections were deparaffinized in xylene and rehydrated in a graded alcohol series and boiled with citrate buffer pH 6. Sections were incubated with primary antibodies specific for NNMT or ki67 All biopsy specimens from patients with NSCLC were stained with hematoxylin and eosin and NNMT and reviewed by the board-certified pathologists in the Department of Pathology at Hospital Quirónsalud (Valencia, Spain). For antibody detection, Envision detection system peroxidase/DAB (Dako) was used. The procedure was performed on a Dako Autostainer Link 48. Image acquisition and analyses were performed with a Leica DMD108 microscope. The NNMT staining score were developed by the board-certified pathologists and designated as follows: -, negative expression; +, weak expression; ++, moderate expression; +++, strong expression.

Lung tissue samples from idiopathic fibrotic patients were fixed in 10% formalin neutral buffer solution for 24 h, and then embedded in paraffin before being cut into 4-µm-thick sections.

For histological examination, sections were stained with Mayer's hematoxylin and then with 1% eosin alcohol solution (H&E staining). For staining of collagen (Masson's trichrome staining), sections were treated sequentially with solution A (5% w/v potassium dichromate and 5% w/v TCA). And for immunohistochemical analysis of α-SMA, samples were incubated 12 h with an antibody against α-SMA from Abcam.

### Example 1.15. Biopsies from patients

This study was carried put in accordance with the Declaration of Helsinki including all amendments and participating hospitals required Institutional Research Ethics Board approval. All patients provided their written informed consent prior to being included in the study and before study procedures, sampling, and analyses. Following routine clinical practice, patients' data were retrospectively obtained from patients' medical records. All 24 EGFR-mutant NSCLC patients who progressed on EGFR TKI therapy underwent second biopsy of their tumor at Hospital Clinico Universitario and Hospital Universitario La Fe in Valencia, Spain and were included in the study cohort. Samples used for analyses had to have both pre- and posttreatment tumor specimens available. The medical records were reviewed retrospectively by board-certified physicians to obtain clinical information under an Institutional Review Board-approved protocol. All relevant ethical regulations were followed. The mutational status of EGFR (both primary and secondary mutations) was determined by the institutions as part of their diagnostic routine.

### Example 1.16. Murine drug treatment studies

All animal treatment studies were reviewed and approved by the Institutional Animal Care and Use Committee at University of Valencia. Approximately 2×10⁶ total cells were injected subcutaneously in the flanks of NOD *scid* gamma (NSG) mice. The size of the engrafted tumors was measured with digital calipers and grown until approximately 100 mm³. The tumors were then randomized, and mice were treated (intraperitoneally) daily for 20 days either with vehicle or daporinad (10 mg/kg).

Immunohistological analysis of mouse xenografts was performed similarly to samples from EGFR-mutant NSCLC patients.

### Example 1.17. Immunofluorescence

Briefly, 0,1×10⁶ cells were plated in 12 well plates and left 24 hours to adhere. After 24 hours cells were fixed in 4% PFA. After permeabilization with 0.2% Triton X-100 cells were blocked. Primary antibodies were incubated overnight in a wet-chamber and the conjugated secondary was incubated at room temperature for one hour. Nuclei were labelled with Hoechst 33342 (Molecular Probes, Life Technologies). Image acquisition and analyses were performed with the Olympus FV1000 confocal microscope with a 40X objective.

### Example 1.18. Predictive Modeling

A total of 69 patients were stratified into good-responders with overall survival (OS) longer than 24 months and poor-responders with OS shorter than 24 months. At baseline, the serum samples were collected from patients for targeted metabolites profiling analysis. Categorical variables associated for each patient (sex, smoking status or TNM staging) were recategorized into binary components.

We developed machine learning-based risk prediction models applying the Gradient Boosting Model (GBM) algorithm with k-fold cross-validation (k = 5) performed on Python 3.7.3 using all analyzed metabolites. After, we developed new models including demographic, clinical, genetic characteristics and metabolites.

Importance score of each feature was automatically calculated. A higher importance score indicated that the variable had a higher predictive value for the model.

The classification quality was evaluated using a comparison of real and the predicted class for each patient. The metrics were ROC curves to obtain Area Under ROC Curve (AUC), sensitivity and specificity and Kaplan-Meier curves.

### Example 2. Results

### Example 2.1 Increased NNMT expression and activity induce mesenchymal phenotype and correlate with poor prognosis and resistance to cancer therapy

The development of irreversible EGFR TKIs has provided treatment options to the NSCLC patients with acquired T790M mutations following reversible EGFR TKI treatment; however, NSCLC patients with acquired EGFR TKI resistance with an EMT phenotype do not benefit from targeted therapies. To find the Achilles' heel that can be targeted to destroy the subset of EGFR TKI resistant NSCLC with an EMT phenotype, we interrogated public databases of lung tumour samples for targets that correlates with mesenchymal-specific genes and the EMT signatures we derived from the EGFR TKI resistant NSCLC cells. Among the target candidates, *NNMT* (Nicotinamide N-methyl transferase) positively correlated with mesenchymal genes and negatively correlated with epithelial genes **(****Figure 1B**). Additionally, NNMT significantly associates with EMT signatures unique to EGFR TKI resistant cells. NNMT is an enzyme that transfers methyl groups from the universal donor S-adenosylmethionine (SAM) to nicotinamide (NAM) producing 1-MNA. This use of SAM reduces histone methylation potential, promoting gene expression changes in cancer cells. To investigate if the *NNMT* expression determines clinical outcome, we measured *NNMT* expression in tumors and in the adjacent normal tissue from NSCLC patients with various genotypes **(****Figure 1A** **&** **5B****).** In the cohort, tumors with elevated *NNMT* expression levels compared to the normal adjacent tissues had statistically worse overall survival (p=0.0454) than patients with lower NNMT expression in their tumors **(****Figure 2****).** We then sought to measure serum 1-MNA levels as a surrogate marker of NNMT activity. Using UPLC-MS, we measured 1-MNA level in serum samples from *EGFR* mutant NSCLC patients undergoing EGFR TKI therapies **(****Figure 14****).** The patients with higher serum 1-MNA level had worse OS than the patients with lower serum 1-MNA level **(****Figure 14****).** Based on the strong correlation between the EMT and CDH1KD signatures derived from *EGFR* mutant NSCLC cells grown resistant to EGFR TKI **(****Figure 1B**), we sought to investigate consecutive formalin-fixed paraffin-embedded (FFPE) tissue samples from advanced stage EGFR-mutant NSCLC patients who radiographically progressed on EGFR TKI treatment for the presence of a T790M secondary mutation and the overexpression of NNMT **(****Figure 5B****).** 13 of the 22 post-EGFR TKI specimens (59.1%) harboured a T790M mutation. Board-certified pathologist scored IHC samples for NNMT protein expression and 6 of the 22 (27.3%) patients showed increased NNMT expression at the time of disease progression; of these 6 patients, 5 patients (83.3%) did not have concurrent T790M secondary mutation and only 1 of 13 patients (7.7%) who harboured a T790M secondary mutation had increased NNMT level **(****Figure 5A & Figure 5B****).**

To investigate whether *NNMT* expression correlates with the expression of mesenchymal and epithelial genes in NSCLC cell lines, we queried the gene expression data of the CCLE database. A significant positive correlation of *NNMT* expression with mesenchymal genes and a negative correlation with epithelial -specific genes was found **(****Figure 1B**). The EMT and CDH1KD signatures derived from EGFR mutant NSCLC cells also strongly correlates with *NNMT* expression.

To test if the induction of a mesenchymal phenotype in EGFR mutant NSCLC cells is sufficient to promote the overexpression of NNMT, we induced a mesenchymal phenotype in otherwise epithelial EGFR mutant NSCLC by chronically expose the cells to TKIs or TGFβ1 or depleting E-cadherin (1-3). As we previously have shown (3), the induction of a mesenchymal phenotype in EGFR mutant NSCLC promotes EGFR TKI resistance **(****Figure 4A** **&** **4B****).** Considering that the induction of mesenchymal phenotype is epigenetically regulated, we compared global transcriptomic, proteomic and epigenetic changes in three parentals (i.e. HCC827, HCC4006, H1975) EGFR mutant NSCLC against their respective EGFR TKI resistant cell lines (i.e. ERC4, HCC4006Ge-R, H1975O-R) with a mesenchymal phenotype derived from the parental cell lines. To easy compare the data, all the changes are represented as median of log2ratio. We compared log2 rations of gene expression (X axis), protein expression (Y axis) and the promoter methylation score (color scale in the secondary Y axis). *NNMT* appears in the upper-right quadrant close to a set of genes with promoter hypomethylation resulting in the robust mRNA expression and protein expression (p<0,05, comparing all 3 pairs, **Figure 4A****).** In our EGFR TKI resistant EGFR mutant NSCLC cells, NNMT is overexpressed in the cells presenting E-cadherin low and vimentin high expressions, a feature common among the mesenchymal cells **(****Figure 4C****).** In contrast, EGFR TKI resistance by MET amplification (ERC13, **Figure 4B****)** or T790M in EGFR mutant NSCLC cells that maintain epithelial phenotype did not increase the NNMT expression. It is during the acquisition of EGFR TKI resistance in HCC4006 cells, that NNMT is upregulated concomitantly to the loss of E-cadherin expression and the increase of vimentin and N-cadherin expression **(****Figure 4D****).** Previously, we have shown that TGFβ1-induced EGFR TKI resistance and the mesenchymal phenotype are reversible in EGFR mutant NSCLC cells by culturing the cells in the absence of TGFβ1, and that the TGFβ1-induced NNMT expression was also reversible **(****Figure 4E****).** Furthermore, the induction of a mesenchymal phenotype by the depletion of CDH1 also resulted in an increase in NNMT expression **(****Figure 4E****).** In summary, we discovered that NNMT expression is a unique event in EGFR TKI resistant EGFR mutant NSCLC cell lines with a mesenchymal phenotype.

Furthermore, this NNMT increasing has been also observed in paraffin-embedded human idiopathic pulmonary fibrosis patients, suggesting that NNMT is important in the pathogenesis of IPF, modulating EMT process **(****Figure 3****).**

To investigate if NNMT is necessary to maintain the EMT-mediated EGFR TKI resistant phenotype, we repressed *NNMT* by shRNA in three EGFR TKI-resistant mesenchymal cell lines (HCC4006Ge-R, H1975O-R and PC9O-R) and their respective parental cells. NNMT repression in mesenchymal cell lines transformed the cells to a more epithelial-state as determined by CDH1 re-expression and down-regulation of other canonical mesenchymal markers (VIM, CDH2, ZEB1) **(****Figure 6A** **&** **6B****).**

To investigate if NNMT is enough for the induction of a mesenchymal phenotype, NNMT was ectopically overexpressed in EGFR TKI-sensitive cells (HCC4006, H1975 and PC9) and changes in EMT phenotype were investigated with confocal microscopy **(****Figure 6B****).** In NNMT overexpressing cells, vimentin intermediate filaments are detected in the cytoskeleton and CDH1 cell-cell junctions are reduced. Lysates from the NNMT overexpressing cells were resolved on western blot to show no apparent changes in canonical markers for EMT **(****Figure 6D****).** These results indicate that NNMT is necessary for the maintenance but not enough for the induction of the mesenchymal phenotype.

Next, we assessed if the NNMT expression affects EGFR TKI sensitivity. Depleting NNMT in the mesenchymal EGFR TKI-resistant cells increases cell death significantly following EGFR TKI treatment **(****Figure 6E****),** decreases the IC50 for EGFR TKIs **(****Figure 7A****)** and reducing the number of surviving colonies after long-term treatment with EGFR TKI **(****Figure 7E****).**

Although overexpressing NNMT did not affect phenotype, NNMT overexpressing cells increase EGFR TKI resistance, reducing significantly cell death after short term drug treatment **(****Figure 6F****)** and increasing the number of surviving colonies **(****Figure 7E****).** In HCC4006 cells, NNMT is necessary to promote the EGFR TKI resistant phenotype which is further enhanced with EGFR TKI treatment (100 nM, 30 days). **(****Figure 7F****).** This increased sensitivity to EGFR TKIs after NNMT repression could be explained to a regained EGFR signaling addiction that EGFR TKI treatment can effectively shut down by a decreased phosphorylation in EGFR/Akt/ERK/S6 signaling pathway **(****Figure 7C** and **7D****).**

In summary, we find that increased NNMT expression and activity is present in the induction of EMT process (in cancer and other pathologies) and significantly correlated with poor prognosis and therapy's resistance.

### Example 2.2. Increased expression of NNMT alters NAD⁺ metabolism in EGFR mutant NSCLC cells, determining a new vulnerability based on NAD⁺ depletion

NNMT transfers methyl groups from the universal donor SAM to NAM, producing 1-MNA regulating the availability of NAM for NAD⁺ synthesis **(****Figure 8A****).** Based on the discovery that NNMT expression is increased in EGFR TKI-resistant NSCLC cells, we hypothesized that NNMT metabolites from the methionine and NAD⁺ pathways could be altered in EGFR TKI resistant cells with a mesenchymal phenotype. The level of 1-MNA, which is a metabolite of NAM produced by NNMT, is significantly increased in EGFR mutant cells grown resistant to EGFR with a mesenchymal phenotype **(****Figure 8A****).** Similarly, the levels of NAM, which is a substrate of NNMT, are statistically lower in EGFR TKI-resistant cells **(****Figure 8B****).** The increased NNMT activity promoted an increased production of 1-MNA resulting in reduced NAD⁺ levels in EGFR TKI-resistant cells **(****Figure 8C****).** Finally, we measured the SAM/SAH ratio to evaluate SAM utilization, that are lower in cell lines with high NNMT than in cell lines with low NNMT **(****Figure 8D****).** The increased use of SAM in mesenchymal cells has to be replenished by methionine leads to reduced levels of this metabolite **(****Figure 9A****).** The polyamine synthesis pathway is essential for cell growth and proliferation and consumes SAM producing 5-MTA. We didn't find significant changes in 5-MTA **(****Figure 9B****)** confirming that in our models, SAM/SAH lower ratio levels are not due to an increase in this pathway. Lastly, a direct consequence of lower SAM/SAH ratio in NNMT^{high} cells is a reduction in glutathione levels **(****Figure 9H****).**

Since NAD⁺ metabolism is dysregulated in EGFR TKI-resistant cells with a mesenchymal phenotype, we hypothesized that the resistant cells adapt their metabolism depending on NNMT activity.

Overexpressing NNMT in an EGFR TKI-sensitive cell line (HCC4006) or depleting NNMT in EGFR TKI resistant mesenchymal cells (HCC4006Ge-R) results in a deep metabolism rewiring. First, 1-MNA, NNMT sub-product, is detected 3,000 times more in NNMT overexpressing cells (HCC4006NNMT and HCC4006Ge-R shNT). Second, SAM/SAH ratio and NAM, surrogate markers of NNMT activity decreased when NNMT levels are higher in the cells. Finally, NAD⁺ levels decreased in the cells with NNMT overexpression and depletion of NNMT restored NAD⁺ levels **(****Figure 10A& 10B**).

NAD⁺ is an essential co-factor in several metabolic processes therefore its levels are highly regulated. Among the three NAD⁺ salvage pathways, NAMPT catalyzes the main reaction using NAM as substrate to produce NAD⁺ **(****Figure 11A****).** FK866/APO866, daporinad (DAP) is a potent and specific NAMPT inhibitor, which is being used in different cancer clinical trials, that compromises the pool of cellular NAD⁺ (von Heideman, 2010; Goldinger 2016). EGFR TKI-resistant cells lead NAM to produce 1-MNA instead of NAD⁺ which prompted us to investigate a potential selective vulnerability of EGFR TKI-resistant cells to NAD⁺ depletion. Genetically repressing NAMPT reduces viability almost up to 50% in gefitinib resistant HCC4006Ge-R cells **(****Figure 12B****).** DAP treatment of NNMT overexpressing cells shows that mesenchymal cells are exceptionally sensitive to this inhibitor **(****Figure 11B****).** The anti-tumor effect seen in NNMT^{high} cells is, as expected due to a drop in NAD⁺ levels that can be rescued with nicotinamide mononuclueotide (NMN), NAMPT product **(****Figure 11C****)** reducing cell death induced by DAP **(****Figure 11D****).** To confirm that DAP targets selectively NNMT^{high} cells by an energetic collapse due to a drop in NAD⁺ levels, we measured NAMPT mRNA levels in HCC4006 and HCC4006Ge-R cells. Although NAMPT mRNA is lower in HCC4006Ge-R cells, overexpressing NAMPT **(****Figure 12C****)** does not affect NNMT expression **(****Figure 12D****)** or change DAP sensitivity **(****Figure 12E****)** indicating that EGFR TKI-resistant cells are specifically sensitive to DAP not due to NAD⁺ synthesis deficiencies but because the increased NNMT activity compromises NAD⁺ levels.

We confirmed that NNMT overexpressing cells are remarkably sensitive to NAMPT inhibition and repressing NNMT in resistant HCC4006Ge-R cells reduces significantly DAP-induced cell death **(****Figure 11E & 11F****).** Moreover, overexpressing NNMT in epithelial HCC4006 cells is enough to sensitize cells to DAP treatment **(****Figure 12F****).** High sensitivity to DAP is also extrapolated to alternative forms of EMT activation with increased NNMT expression, like chronic TGFβ1 exposure or stable CDH1 repression cell models **(****Figure 12G****).**

Although DAP severely drops NAD⁺ levels but inhibiting the main NAD⁺ consuming enzymes with olaparib (PARP) or selisistat (sirtuins) does not prevent DAP cytotoxic effect. Only pharmacological or genetic inhibition of NNMT can revert it **(****Figure 11G****),** suggesting that this cytotoxic effect of DAP on mesenchymal EGFR TKI-resistant cells is NNMT dependent.

In summary, we find that NNMT activity correlates with metabolites from methionine cycle, trans-sulfuration pathway. cystine/cysteine cycle, polyamine pathways and NAD+ salvage cycle, specially, NNMT activity direct product 1-MNA. This phenomenon is also observed in TKI-resistance cells and NSCLC patients.

NNMT contributes to NAD+ pathway homeostasis, so an NAD+ production inhibitor as NAMPT inhibitor, could generate a vulnerability specific to cells overexpressing NNMT, reducing EMT process and improving prognosis and reducing the acquisition of resistance against therapies.

On the other hand, identification of patients with high levels of NNMT could serve to predict the response to NAMPT inhibitors.

### Example 2.3. Therapies directed against NAD + and methionine metabolism, such as NNMT or NAMPT inhibition, contribute to promote mesenchymal to epithelial transition and to ameliorate EGFR TKI sensitivity

Since the accumulation of 1-MNA limits NNMT enzymatic activity, inhibitory activity of other small N-methylated molecules has been studied, including 1-methylquinolinium (1-MQ). Considering the increased NNMT activity in EGFR TKI resistant cells with a mesenchymal phenotype, we hypothesized that pharmacological inhibition of NNMT with 1-MQ could mimic the depletion of *NNMT* using shRNA. We sought that inhibition of NNMT activity with 1-MQ in mesenchymal resistant cells could prevent DAP cytotoxic effect. Treatment of mesenchymal EGFR TKI resistance cells with 1-MQ resulted in a significant decrease in 1-MNA level that indirectly suggests a decreased NNMT activity **(****Figure 13A****).** 1-MQ treatment abolishes completely DAP cytotoxicity in mesenchymal cells **(****Figure 13B****).** This reduction in cell death can be explained because 1-MQ treatment prevented the abrupt drop of NAD⁺ levels **(****Figure 13C****)** following DAP treatment in EGFR TKI-resistant NNMT^{high} cells. Long-term treatment of EGFR TKI-resistant cells with 1-MQ promotes the mesenchymal-to-epithelial transition, increasing CDH1 cell-to-cell junctions and reducing vimentin levels **(****Figure 13D****).** The transition to the epithelial state by 1-MQ treatment associates with an increased EGFR TKI sensitivity **(****Figure 13E & 13F****)** similar to the depletion of NNMT by shRNA **(****Figure 7E & 7E****).**

We have previously shown that NNMT expression is increased during EGFR TKI resistance **(****Figure 4D****)** and also that 1-MQ can inhibit NNMT favoring a more pronounced epithelial phenotype. Thus, we hypothesized that the small molecule 1-MQ could prevent or delay EGFR TKI-acquired resistance. As expected, stably expressing an active NNMT protein allows cells to acquire osimertinib resistance faster than those cells with mutated NNMT protein. In both cell lines, pharmacological inhibition of NNMT with 1-MQ prevents the appearance of drug resistant cells **(****Figure 13G****).**

Motivated by the results that the EGFR TKI-resistant tumors with a mesenchymal phenotype is sensitive to single-agent DAP treatment *in vitro* **(****Figure 11B****),** we sought to test the impact of combination of NNMT and NAMPT. We observed that combination of both inhibitors reduced aggressiveness and drug resistance for TKI therapies **(****Figure 12B****).**

In summary, we find that inhibition of NNMT activity combined with TKI presents improved results than the use of TKI alone. And given that NNMT increase correlates with NAD+ dependency, combination of NNMT inhibitors with inhibitors of the production of this metabolite, such as NAMPT inhibitors, considerably reduce the acquisition of resistance to TKIs.

### Example 2.4. Detection of NNMT related metabolites to predict survival of NSCLC's patients treated with TKIs

Since NNMT could be a useful therapeutic target against different diseases, we set out to identify biomarkers of its activity. We have observed that certain metabolites related to NNMT activity, such as S-Adenosyl methionine (SAM), 5'-methylthioadenosine (5-MTA), Methionine, Cystine, Nicotinamide, Spermidine, Spermine, Cystathionine, Cysteine, HomoCysteine, Glutamyl-L- cysteine (GluCys), S-adenosyl-L-homocysteine (SAH), Glutathione Oxidized (CSSG), Glutathione (GSH), Glutathione disulfide (GSSG), or NAD+ are altered in cell lines that have acquired resistance to therapy against TKI **(****Figure 8** **&** **9****).** We have also been able to observe that the levels of these metabolites are directly related to NNMT activity, performing cell models with down-expression of NNMT **(****Figure 10A****)** or with over-expression **(****Figure 10B****).**

We have observed a good correlation between serum level of NNMT metabolite, *1-MNA*, and progression free survival (PFS) or Overall survival (OS) in a cohort of 69 EGFR mutant NSCLC patients. Patients with high levels of 1MNA, representative of high NNMT activity, had worse survival than those patients with low levels of 1MNA **(****Figure 14A****).** 1-MNAhigh/low stratified according to above or below the median value respectively. Similar results were observed with other metabolites related to NNMT activity, such as SAH, Spermine or Spermidine, taking as cut-off point the mean instead of the median of each of these metabolites **(****Figure 17****).**

In order to improve the predictive capacity for response to TKI therapy, we designed different machine learning models based on gradient boosting machine 24 months survival after TKI treatment in a cohort of 69 EGFR mutant NSCLC patients. We observed that with the combination of these 15 metabolites we improved the predictive capacity compared to the individual analysis of the metabolites **(****Figure 20****).**

The best of the models obtained a predictive capacity of 0.83 for the AUC (Area Under the Curve) ROC (Receiver Operating Characteristics) curve with a 0.84 for Sensitivity and 0.8 for Specificity **(****Figure 19A****).** With this model, we classified patients as poor or good responders for TKI therapy and generated a Kaplan-Meier curve for OS, demonstrated that the model had high clinical utility. Our GBM model was able to stratify patients with a p-value <0.005 (Log-Rank test) **(****Figure 19B****).**

But we were also able to verify that if we add demographic information such as age and gender to the information provided by these biomarkers, or if the patient is a smoker or not, we can further improve the ability to predict the response to TKI therapy.

Our predictive model associated to 2-years overall survival obtained a p value <0.005 in Kaplan-Meier analysis **(****Figure 21B****)** with high accuracy (AUC: 0.94; Accuracy: 0.97; Sensitivity: 1.0; Specificity: 0.88) **(****Figure 21A****).**

The results confirmed a high discriminatory power of blood NNMT related metabolites to distinguish between good and poor responders against TKI therapy in NSCLC. These data indicate that our method using mass spectrometry to measure blood NNMT related metabolites and machine learning models is a promising tool that could be used to support a better management of cancer's patients. Despite the fact that these results have been obtained in a small cohort of patients, so it should be validated in a larger number of patients, it should be noted that the models obtained are based on machine learning strategies that allow the improvement of performance with adding new data.

In summary, using mass-spectrometry quantification of NNMT related metabolites and machine learning models we can identify which patients present an increase in NNMT levels and therefore a weak response to TKI treatment, resulting in a low survival compared to those patients with low NNMT levels and a better response to TKIs. This method could be used in clinical practice for the rapid and accurate identification of patients with a high risk of progression who may benefit from more aggressive treatment and may improve therapeutic strategies for patients with NSCLC.

## Claims

1. *In vitro* method for assessing the activity of nicotinamide N-methyltransferase (NNMT) in a biological sample which comprises:
a. Determining the concentration level of at least a metabolite selected from the group comprising: 1-MNA, 5-MTA, CSSG, Cystathionine, Cysteine, Cystine, GluCys, GSH, Homocysteine, Methionine, Nicotinamide, SAH, SAM, Spermidine and/or Spermine, or any combination thereof;
b. Wherein the identification of a concentration level of 1-MNA, Cystathionine, Cystine, Homocysteine, Nicotinamide, SAH, SAM, Spermidine and/or Spermine higher than a pre-established threshold level is an indication of high NNMT activity; or wherein the identification of a concentration level of 1-MNA, Cystathionine, Cystine, Homocysteine, Nicotinamide, SAH, SAM, Spermidine and/or Spermine lower than a pre-established threshold level is an indication of low NNMT activity; and
c. Wherein the identification of a concentration level of 5-MTA, CSSG, Cysteine, GluCys, GSH and/or Methionine lower than a pre-established threshold level is an indication of high NNMT activity; or wherein the identification of a concentration level of 5-MTA, CSSG, Cysteine, GluCys, GSH and/or Methionine higher than a pre-established threshold level is an indication of low NNMT activity.

2. *In vitro* method for selecting patients who may benefit from a treatment with NNMT inhibitors which comprises assessing the NNMT activity following the method of claim 1, wherein the identification of high NNMT activity is an indication that the patients may benefit from a treatment with NNMT inhibitors.

3. *In vitro* method for the prognosis and/or predicting the response of patients to NNMT inhibitors which comprises assessing the NNMT activity following the method of claim 1, wherein the identification of high NNMT activity is an indication of poor prognosis and/or that the patients may response to a treatment with NNMT inhibitors.

4. *In vitro* method for predicting whether a patient is resistant to a treatment with tyrosine kinase inhibitors which comprises the identification of high NMMT activity following the method of claim 1.

5. *In vitro* method for screening NNMT inhibitor candidates which comprises:
a. Determining the concentration level of at least a metabolite selected from the group comprising: 1-MNA, 5-MTA, CSSG, Cystathionine, Cysteine, Cystine, GluCys, GSH, Homocysteine, Methionine, Nicotinamide, SAH, SAM, Spermidine, and/or Spermine, or any combination thereof, after administering the candidate NNMT inhibitor;
b. Wherein the identification of a concentration level of 1-MNA, Cystathionine, Cystine, Homocysteine, Nicotinamide, SAH, SAM, Spermidine and/or Spermine lower than a pre-established threshold level; or wherein the identification of a concentration level of 5-MTA, CSSG, Cysteine, GluCys, GSH and/or Methionine higher than a pre-established threshold level, is an indication of the candidate is a NNMT inhibitor.

6. *In vitro* method, according to any of the previous claims, which comprises determining the concentration level of at two metabolites selected from the group: 1-MNA, 5-MTA, CSSG, Cystathionine, Cysteine, Cystine, GluCys, GSH, Homocysteine, Methionine, Nicotinamide, SAH, SAM, Spermidine and/or Spermine.

7. *In vitro* method, according to any of the previous claims, which comprises determining the concentration level of at least the following two metabolites: 1-MNA and 5-MTA, 1-MNA and SAM, and/or 5-MTA and SAM

8. *In vitro* method, according to any of the previous claims, which comprises determining the concentration level of at two metabolites selected from the group: 1-MNA, 5-MTA, CSSG, Cystathionine, Cysteine, Cystine, GluCys, GSH, Homocysteine, Methionine, Nicotinamide, SAH, Spermidine and/or Spermine; in combination with SAM

9. *In vitro* method, according to any of the previous claims, which comprises determining the concentration level of at least the following three metabolites: 1-MNA, 5-MTA and SAM

10. *In vitro* method, according to any of the previous claims, wherein the patient is suffering from lung cancer, preferably from Non-small cell lung cancer, and most preferably from Non-small cell lung cancer with EGFR mutation.

11. *In vitro* method, according to any of the previous claims, wherein the biological sample obtained from the patients is serum, blood, plasma, sputum, biopsy, urine or cell cultures or cell supernatants.

12. *In vitro* method, according to any of the previous claims, wherein the method is a mass spectrometry-based method.

13. *In vitro* use of at least a metabolite selected from the group comprising: 1-MNA, 5-MTA, CSSG, Cystathionine, Cysteine, Cystine, GluCys, GSH, Homocysteine, Methionine, Nicotinamide, SAH, SAM, SAM, Spermidine and/or Spermine, or any combination thereof, for assessing NNMT activity, for selecting patients which may benefit from a treatment with NNMT inhibitors, for the prognosis and/or predicting the response of patients to NNMT inhibitors, for predicting whether a patient is resistant to a treatment with tyrosine kinase inhibitors and/or for screening NNMT inhibitor candidates.

14. NNMT inhibitors for use in the treatment of patients, wherein the biological sample obtained from the patients is **characterized by** a concentration level of:
a. 1-MNA, Cystathionine, Cystine, Homocysteine, Nicotinamide, SAH, SAM, Spermidine and/or Spermine higher than a pre-established threshold level; or
b. 5-MTA, CSSG, Cysteine, GluCys, GSH and/or Methionine lower than a pre-established threshold level.
